# FASCICULE DE BREVET EUROPEEN

(11) **EP 1 078 094 B1**
(45) Date de publication et mention de la délivrance du brevet: **07.07.2010**
(21) Numéro de dépôt: 99920883.8
(22) Date de dépôt: 18.05.1999
(51) Int. Cl.: C12N 15/86, C12N 5/10, C12N 7/00

(54) **METHODES ET COMPOSITIONS POUR LA PRODUCTION DE PARTICULES VIRALES**
METHODEN UND ZUSAMMENSETZUNGEN FÜR DIE PRODUKTION VON VIRUSPARTIKELN
METHODS AND COMPOSITIONS FOR PRODUCING VIRAL PARTICLES

(30) Priorité: 18.05.1998 FR 9806258
(43) Date de publication de la demande: 28.02.2001
(73) Titulaire: Aventis Pharma S.A., 92160 Antony (FR); GENOPOIETIC, 01700 Beynost (FR)
(72) Inventeur: TORRENT, Christophe, F-75016 Paris (FR); YEH, Patrice, F-75005 Paris (FR); PERRICAUDET, Michel, F-28320 Ecrosnes (FR); KLATZMANN, David, F-75013 Paris (FR); SALZMANN, Jean-Loup, F-75005 Paris (FR)
(74) Mandataire: Becker, Philippe
(86) Numéro de dépôt international: PCT/FR1999/001184
(87) Numéro de publication internationale: WO 1999/060144

(56) Documents cités:
- WO-A-97/32481
- WO-A-98/22143
- BILBAO G. ET AL.: "Adenoviral/retorviral vector chimeras: a novel strategy to achieve high-efficiency stable transduction in vivo." FASEB JOURNAL, vol. 11, 1997, pages 624-634, XP002091318
- MILLER N ET AL: "TARGETED VECTORS FOR GENE THERAPY" FASEB JOURNAL, vol. 9, no. 2, février 1995 (1995-02), pages 190-199, XP000616414
- DEDIEU J.-F. ET AL.: "Long-term gene delivery into the livers of immunocompetent mice with E1/E4-defective adenovirus." JOURNAL OF VIROLOGY, vol. 71, no. 6, juin 1997 (1997-06), pages 4626-4637, XP002091949
- GAO G.-P. ET AL: 'Biology of adenoviral vectors with E1 and E4 deletions for liver-directed gene therapy' JOURNAL OF VIROLOGY vol. 70, no. 12, Décembre 1996, pages 8934 - 8943
- BROUGH D.E. ET AL: 'Activation of transgene expression by early region 4 is responsible for a high level of persistent transgene expression from Adenovirus vectors in vivo' JOURNAL OF VIROLOGY vol. 71, no. 12, Décembre 1997, pages 9206 - 9213
- FENG M. ET AL: 'Stable in vivo gene transduction via a novel adenoviral/ertroviral chimeric vector' NATURE BIOTECHNOLOGY vol. 15, Septembre 1997, pages 866 - 870
- TORRENT C. ET AL: 'Transgene amplification and persistence after delivery of retroviral vector and packaging functions with E1/E4-deleted adenoviruses' CANCER GENE THERAPY vol. 7, no. 8, 2000, pages 1135 - 1144

### LISTE DE SEQUENCES

<110> RHÔNE-POULENC RORER S.A. GENOPOIETIC SARL
<120> METHODES ET COMPOSITIONS POUR LA PRODUCTION DE PARTICULES VIRALES
<130> B3946A - PB/KM
<140>
   <141>
<150> 9806258
   <151> 1998-05-18
<160> 16
<170> PatentIn Ver. 2.1
<210> 1
   <211> 36
   <212> ADN
   <213> Séquence artificielle
<220>
   <223> Description de la séquence artificielle: OLIGONUCLEOTIDE
<400> 1
   ggctctagag ccgccaccat ggcgcgttca acgctc 36
<210> 2
   <211> 39
   <212> ADN
   <213> Séquence artificielle
<220>
   <223> Description de la séquence artificielle: OLIGONUCLEOTIDE
<400> 2
   aaaaaaaagc ggccgcttat catggctcgt actctatgg 39
<210> 3
   <211> 30
   <212> ADN
   <213> Séquence artificielle
<220>
   <223> Description de la séquence artificielle: OLIGONUCLEOTIDE
<400> 3
   caggcatgga gtcaaaacca gagcctggac 30
<210> 4
   <211> 25
   <212> ADN
   <213> Séquence artificielle
<220>
   <223> Description de la séquence artificielle: OLIGONUCLEOTIDE
<400> 4
   cactgcctta atcaaaaccc agcag 25
<210> 5
   <211> 36
   <212> ADN
   <213> Séquence artificielle
<220>
   <223> Description de la séquence artificielle: OLIGONUCLEOTIDE
<400> 5
   ccggaattcg ccgccaccat gggccagact gttacc 36
<210> 6
   <211> 20
   <212> ADN
   <213> Séquence artificielle
<220>
   <223> Description de la séquence artificielle: OLIGONUCLEOTIDE
<400> 6
   ggaggcggag gcttagggtg 20
<210> 7
   <211> 20
   <212> ADN
   <213> Séquence artificielle
<220>
   <223> Description de la séquence artificielle: OLIGONUCLEOTIDE
<400> 7
   gtagacggca tcgcagcttg 20
<210> 8
   <211> 36
   <212> ADN
   <213> Séquence artificielle
<220>
   <223> Description de la séquence artificielle: OLIGONUCLEOTIDE
<400> 8
   aaaaaaaagc ggccgctcat tagggggcct cgcggg 36
<210> 9
   <211> 21
   <212> ADN
   <213> Séquence artificielle
<220>
   <223> Description de la séquence artificielle: OLIGONUCLEOTIDE
<400> 9
   gaccccacct gtaggtttgg c 21
<210> 10
   <211> 32
   <212> ADN
   <213> Séquence artificielle
<220>
   <223> Description de la séquence artificielle: OLIGONUCLEOTIDE
<400> 10
   aaaaaaaagc ggccgctgca actgcaagag gg 32
<210> 11
   <211> 35
   <212> ADN
   <213> Séquence artificielle
<220>
   <223> Description de la séquence artificielle: OLIGONUCLEOTIDE
<400> 11
   aaaaaaaagc ggccgctaaa tgctagagct cgctg 35
<210> 12
   <211> 25
   <212> ADN
   <213> Séquence artificielle
<220>
   <223> Description de la séquence artificielle: OLIGONUCLEOTIDE
<400> 12
   cgcggatccc caccgcatcc ccagc 25
<210> 13
   <211> 25
   <212> ADN
   <213> Séquence artificielle
<220>
   <223> Description de la séquence artificielle: OLIGONUCLEOTIDE
<400> 13
   gctagcttaa gtaacgccat tttgc 25
<210> 14 <211> 22

   <212> ADN
   <213> Séquence artificielle
<220>
   <223> Description de la séquence artificielle: OLIGONUCLEOTIDE
<400> 14
   gcgccaggtc gcatatcgtc gg 22
<210> 15
   <211> 23
   <212> ADN
   <213> Séquence artificielle
<220>
   <223> Description de la séquence artificielle: OLIGONUCLEOTIDE
<400> 15
   gctcgtccgg gatttggaga ccc 23
<210> 16
   <211> 23
   <212> ADN
   <213> Séquence artificielle
<220>
   <223> Description de la séquence artificielle: OLIGONUCLEOTIDE
<400> 16
   gctcgtccgg gatttggaga ccc 23

## Description

La présente invention concerne le domaine des Biotechnologies. Elle concerne plus particulièrement des méthodes et constructions permettant la production de particules rétrovirales, in vitro, ex vivo ou les-dites constructions pour utilisation in vivo. Elle concerne également l'utilisation de ces méthodes et constructions pour le transfert d'acides nucléiques dans les cellules, in vitro, ex vivo, ou les-dites constructions pour utilisation in vivo.

Les rétrovirus constituent aujourd'hui l'un des principaux vecteurs pour le transfert d'acides nucléiques dans les cellules. Ils sont utilisés par exemple pour le transfert d'acides nucléiques in vitro ou ex vivo (expérimentation, étude de régulation, production de protéines recombinantes, introduction de gènes de résistance ou de gène toxiques) ou directement in vivo (création de modèle pathologiques chez l'animal, études de marquage ou de biodisponibilité, utilisation thérapeutique par greffe de cellules productrices ou injection de virus purifiés, etc.).

Pour toutes ces utilisations, il est donc important de disposer de techniques efficaces de production et d'administration de rétrovirus. A cet égard, les techniques utilisées classiquement reposent sur l'emploi de lignées cellulaires d'encapsidation. Ces lignées sont construites in vitro et expriment l'ensemble des protéines nécessaires à la constitution et à l'encapsidation d'un vecteur rétroviral défectif dans une particule rétrovirale. De telles lignées sont par exemples les lignées PSICRIP (Danos et Mulligan, PNAS 85 (1988) p. 6460), PA317 (Miller et Buttimore, Mol. Cell. Biol. 6 (1986) p. 2895), ou GP + Env AM12 (Markowitz et al. Virology 167 (1988) p. 400). Néanmoins, l'emploi de telles lignées peut poser un certain nombre de problèmes, liés tout d'abord à leur construction, ensuite à leur utilisation.

Ainsi, ces lignées doivent être stables, c'est-à-dire exprimer les fonctions rétrovirales de manière permanente, sans réarrangements génétiques ni perte d'expression. De plus, ces lignées doivent être compatibles avec un usage pharmacologique potentiel des rétrovirus, et donc être établies à partir de cellules cultivables, faiblement voire non-immunogènes, etc.

D'autre part, il est important que les lignées utilisées produisent des titres relativement élevés de virus, dépourvus de particules réplicatives contaminantes (RCV). Les procédés de préparation de rétrovirus utilisant des lignées d'encapsidation comportent donc nécessairement des étapes de contrôle de la qualité à la fois des stocks viraux produits et des lignées utilisées. Enfin, selon les applications envisagées, ces productions peuvent devoir être réalisées en respectant des degrés de confinement élevés, ce qui complique encore le caractère industrialisable de ces méthodes.

Afin de remédier à ces inconvénients, il a été proposé dans la demande WO 95/22617 un nouveau concept de production de particules rétrovirales, évitant le recours aux lignées d'encapsidation de rétrovirus. Ce nouveau concept consiste à transformer une cellule cible in vitro, ex vivo ou directement in vivo, en cellule productrice de rétrovirus, en introduisant dans cette cellule l'ensemble des éléments génétiques nécessaires à la constitution d'une particule rétrovirale. Cette demande prévoit en particulier le transfert de ces éléments au moyen de constructions plasmidiques ou de vecteurs viraux d'origine différente. Feng et al. (Nature Biotechnology 15 (1997) p 866) ont par ailleurs décrit un mode particulier de mise en oeuvre de cette nouvelle approche, utilisant deux adénovirus recombinants de première génération (i.e., défectifs pour la région E1), dans lesquels étaient répartis selon un schéma-précis les éléments génétiques rétroviraux.

La présente demande décrit maintenant une nouvelle amélioration aux techniques de production de particules rétrovirales décrites ci-dessus, et sur l'utilisation pour le transfert d'acides nucléiques.

La présente demande repose notamment sur l'utilisation d'adénovirus recombinants pour délivrer à des cellules, in vitro, ex vivo ou in vivo, l'ensemble des éléments génétiques nécessaires à la constitution d'une particule rétrovirale. Par rapport aux travaux décrits antérieurement, la présente demande repose en partie sur l'utilisation de types particuliers d'adénovirus recombinants et sur une répartition particulière des éléments génétiques rétroviraux. Comme démontré dans la présente demande, les méthodes et constructions selon l'invention permettent d'obtenir des taux de production de particules rétrovirales élevés, sont efficaces, présentent une sécurité accrue, aussi bien in vitro, ex vivo que in vivo. En outre, les rétrovirus recombinants défectifs produits sont infectieux, et sont capables de transférer avec une grande efficacité, un acide nucléique dans une cellule.

Un premier objet de l'invention concerne donc une composition comprenant l'ensemble des éléments génétiques nécessaires à la constitution d'une particule rétrovirale, incorporés dans deux adénovirus recombinants comprenant des délétions affectant tout ou une partie fonctionnelle des régions E1, E4 et E3, lesdits adénovirus recombinants comprenant la répartition des éléments génétiques retroviraux telle que décrite dans revendication 1.

Le terme "éléments génétiques nécessaires à la constitution d'une particule rétrovirale" désigne au sens de l'invention l'ensemble des séquences nucléiques, codantes ou régulatrices, qui agissant en cis et en trans sont nécessaires et suffisantes pour constituer une particule rétrovirale, c'est-à-dire une particule physique à la surface de laquelle l'enveloppe rétrovirale est exprimée, et à l'intérieur de laquelle se trouvent les différentes protéines nécessaires à la réalisation d'un cycle rétroviral et un génome sous une forme permettant sa rétrotranscription.

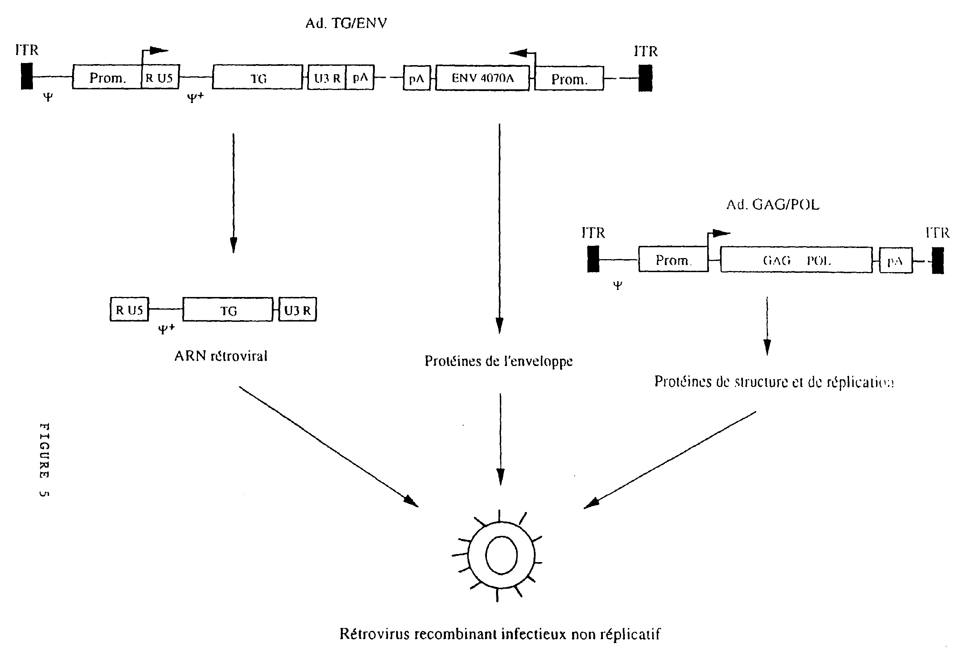

L'organisation génomique des rétrovirus est bien connue, et comprend essentiellement les éléments suivants :
- Une région LTR, localisée à chaque extrémité du génome rétroviral, servant notamment d'origine de transcription et de promoteur transcriptionnel. Cette région LTR se compose plus précisément d'éléments désignés U3, R et U5 (voir Figure 5, par exemple). La séquence U5, conjointement avec la séquence U3, joue un rôle clef lors de l'intégration du provirus.
- Une séquence d'encapsidation (Psi ou ψ), impliquée dans l'encapsidation du génome rétroviral dans la particule virale. La séquence d'encapsidation peut par ailleurs comporter une région étendue à certains éléments du gène gag, qui ont été décrits comme améliorant l'efficacité d'encapsidation. - Trois régions codantes, désignées gag, pol et env, codant respectivement pour les protéines du core (gag), les enzymes (transcriptase inverse, protéase, intégrase) et l'enveloppe du rétrovirus (env).

Pour la constitution d'une particule rétrovirale recombinante, un vecteur rétroviral est généralement construit comprenant les éléments génétiques agissant en cis, c'est-à-dire les régions LTR et la séquence d'encapsidation, et dans lequel tout ou partie des régions codantes gag, pol et/ou env (agissant en trans), sont éliminées. Habituellement, ce vecteur rétroviral comprend également une séquence nucléique d'intérêt (le transgène). Les régions codantes non présentes ou inactives dans le vecteur rétroviral sont apportées en trans (fonctions de complémentation), de manière à pouvoir reconstituer une particule rétrovirale.

Dans la composition de l'invention, les éléments génétiques comprennent donc un vecteur rétroviral et les acides nucléiques codant pour les fonctions de complémentation rétrovirales (c'est-à-dire pour les fonctions défectives dans le vecteur rétroviral, i.e., gag, pol et env).

Les éléments génétiques comprennent :
- un acide nucléique codant pour une protéine rétrovirale gag,
- un acide nucléique codant pour une protéine rétrovirale pol,
- un acide nucléique codant pour une protéine d'enveloppe, par exemple une protéine rétrovirale env, et
- un acide nucléique comprenant, entre deux régions LTR, une séquence d'encapsidation rétrovirale et une séquence nucléique d'intérêt.

Pour la mise en oeuvre de la présente invention, les éléments génétiques peuvent provenir de différents types de rétrovirus, tels que des virus écotropes et/ou amphotropes. Il peut s'agir notamment de rétrovirus appartenant à la famille des oncovirus, des lentivirus ou des spumavirus. Dans la famille des oncovirus, on peut citer notamment les oncovirus lents, non porteur d'oncogène, tels que par exemple MoMLV, ALV, BLV, ou MMTV et les oncovirus rapides, tels que RSV par exemple. Dans la famille des lentivirus, on peut citer par exemple HIV, SIV, FIV ou CAEV.

Par ailleurs, la ou les régions LTR utilisées peuvent être soit des régions LTR rétrovirales complètes, soit des sous-domaines permettant de reconstituer des LTR complets après activité reverse transcriptase. Ainsi, les LTR utilisés peuvent comprendre des délétions de domaines tels que U3 et U5 par exemple. Dans un mode particulier de mise en oeuvre de l'invention, le vecteur rétroviral comprend un LTR 5', dépourvu de région U3 et un LTR 3' dépourvu de région U5. L'emploi de ce type de construction permet notamment de stabiliser le vecteur pendant les étapes de construction et de réduire les risques de recombinaison. Les éléments génétiques peuvent être directement préparés à partir des rétrovirus isolés, selon les techniques connues de l'homme du métier, et/ou synthétisées artificiellement. Ces éléments peuvent être amplifiés en culture, comme décrit dans les exemples. En outre, les acides nucléiques utilisés peuvent être de l'ADN ou de l'ARN.

Dans une variante préférée de réalisation, les éléments génétiques sont tels qu'ils codent pour des protéines gag et pol de rétrovirus et pour une protéine d'enveloppe permettant d'infecter des cellules humaines. Plus préférentiellement, encore, les protéines gag et pol sont des protéines de rétrovirus choisis parmi MoMLV, ALV, BLV, MMTV et RSV, et la protéine d'enveloppe est une protéine de virus choisi parmi A4070, GALV, RD114, VSV-G ou le virus de la rage. Il est entendu que l'invention peut également être mise en oeuvre en utilisant des variants ou mutants de ces protéines, ayant conservé leur activité biologique, ou des protéines chimères ou hybrides permettant de modifier le tropisme de la particule rétrovirale, notamment de lui conférer une spécificité pour des types cellulaires particuliers.

Dans un mode particulier de mise en oeuvre, la protéine d'enveloppe est donc une protéine d'origine virale, permettant aux particules rétrovirales d'infecter les cellules humaines. Il peut s'agir préférentiellement d'une protéine d'un virus tel que A4070, GALV, RD114, VSV-G ou le virus de la rage. Les enveloppes virales préférées sont A4070, GALV ou VSV-G. Les protéines GAG et POL préférées proviennent des rétrovirus MoMLV.

Selon un mode de réalisation particulier de l'invention, les éléments génétiques permettent la constitution d'une particule de lentivirus, et proviennent donc, au moins en partie, de lentivirus, tels que notamment HIV, SIV, FIV ou CAEV. L'utilisation de rétrovirus recombinants préparés à partir de lentivirus a été décrite dans la littérature et offre certains avantages pour le transfert de gènes dans des cellules quiescentes (Poeschla et al., Nature Medicine 4 (1998) 354). La présente invention permet à présent de mettre à profit cette propriété pour générer, dans des conditions améliorées, un transfert de gènes par les lentivirus.

La composition de l'invention comprend l'ensemble des éléments génétiques nécessaires à la constitution d'une particule rétrovirale répartis dans deux adénovirus recombinants distincts avec la répartition particulière suivante des éléments génétiques rétroviraux à savoir:
- un premier adénovirus recombinant comprenant, incorporés dans son génome, un ou plusieurs acides nucléiques codant pour les protéines rétrovirales gag et pol, et
- un deuxième adénovirus recombinant comprenant, incorporés dans son génome, un acide nucléique codant pour une protéine d'enveloppe telle que défini ci-avant, et un acide nucléique comprenant, entre deux régions LTR complètes ou des sous-domaines permettant de reconstituer des LTRs complets après activité reverse transcriptase, une séquence d'encapsidation rétrovirale et une séquence nucléique d'intérêt, lesdit adénovirus recombinants comprenant des délétions affectant tout ou une partie fonctionelle des régions E1, E4 et E3.

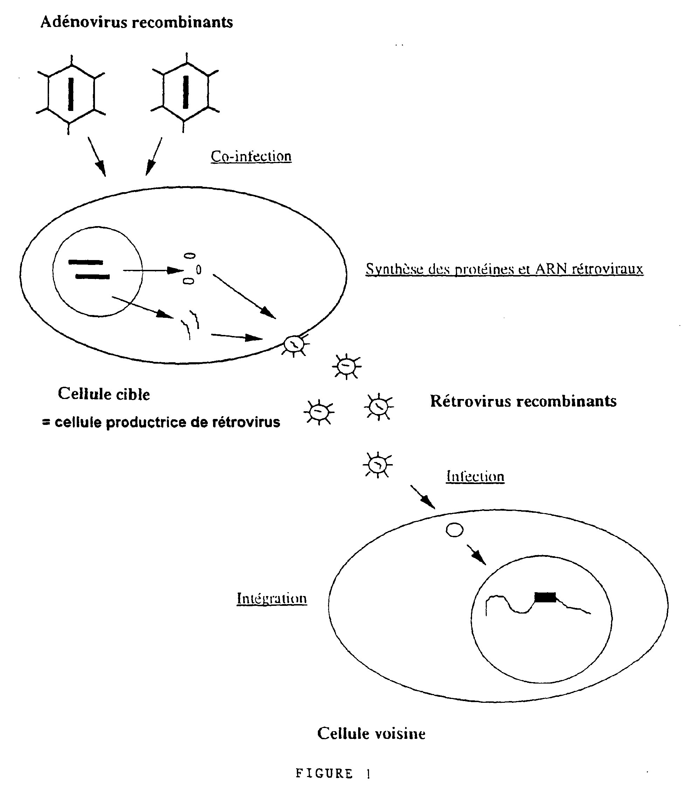

L'utilisation d'une telle composition est illustrée, de manière générale, sur la Figure 1, Comme montré dans les exemples, cette répartition particulière des éléments génétiques rétroviraux permet d'obtenir des titres élevés de particules rétrovirales infectieuses, capables de transférer de manière efficace et stable un transgène dans des cellules désirées.

A cet égard, la présente demande décrit
- tout adénovirus recombinant défectif **caractérisé en ce qu**'il comprend, incorporé dans son génome, un ou plusieurs acides nucléiques codant pour les protéines rétrovirales gag et pol, et
- tout adénovirus recombinant défectif, **caractérisé en ce qu**'il comprend, incorporé dans son génome, un acide nucléique codant pour une protéine d'enveloppe, et un acide nucléique comprenant, entre deux régions LTR complètes ou non, une séquence d'encapsidation rétrovirale et une séquence nucléique d'intérêt.

De tels adénovirus peuvent être défectifs notamment pour tout ou partie de la région E1 (E1A et/ou E1B), E2, et/ou E4, par exemple.

Il est entendu que les compositions décrites peuvent être adaptées par l'homme du métier en fonction de la structure du vecteur rétroviral, et notamment des gènes rétroviraux défectifs dans celui-ci. Ainsi, le vecteur utilisé peut être défectif pour un seul, deux ou l'ensemble des gènes rétroviraux gag, pol et env. Selon le cas de figure, la composition et la répartition des adénovirus dans les compositions de l'invention peut être aisément adaptée. Néanmoins, un mode préféré de réalisation est celui dans lequel le vecteur rétroviral est défectif pour l'ensemble des gènes gag, pol et env, comme illustré dans les exemples et celui adapté à la production de lentivirus recombinants.

Dans les compositions de l'invention, l'acide nucléique utilisé codant pour une protéine rétrovirale gag, pol ou env comprend généralement un promoteur transcriptionnel, situé en 5' de la région codante, et un terminateur transcriptionnel, localisé en 3' de la région codante. Les promoteurs utilisés peuvent être de nature, d'origine et avoir des propriétés variées. Le choix du promoteur utilisé dépend en effet de l'utilisation recherchée et du transgène, notamment. Ainsi, le promoteur peut être un promoteur constitutif ou régulé, fort ou faible, ubiquitaire ou spécifique de tissus/cellules, ou encore spécifique d'états physiologiques ou physiopathologiques (activité dépendante de l'état de différenciation cellulaire ou de certaines étapes du cycle cellulaire). Le promoteur peut être d'origine eucaryote, procaryote, virale, animale, végétale, artificielle ou humaine, etc. Des exemples particuliers de promoteurs sont le promoteur des gènes PGK, TK, GH, EF1-α, APO, CMV, etc. ou des promoteurs artificiels, tels que répondant à p53, E2F ou cAMP.

Préférentiellement, le promoteur contrôlant l'expression de gag, pol et env est un promoteur fort constitutif. En outre, le promoteur peut également comprendre une région "enhancer" permettant d'accroître l'efficacité d'expression. Le choix de la région terminateur peut également être réalisé sans effort par l'homme du métier, notamment parmi les terminateurs des gènes GH, SV40, EF1-α, etc., largement décrits dans la littérature. Enfin, les acides nucléiques codant pour GAG et POL sont souvent utilisés sous forme d'une unité bicistronique, contrôlée par un promoteur et un terminateur uniques, comme c'est le cas dans le génome rétroviral.

Comme indiqué ci-avant, les avantages de la présente invention découlent notamment de la répartition des éléments génétiques rétroviraux dans les adénovirus, et du type d'adénovirus utilisé. A cet égard, comme souligné précédemment, les adénovirus utilisés dans le cadre de la présente invention sont essentiellement défectifs au moins pour la région E1, la région E3 et la région E4, en tout ou en partie. Comme illustré dans les exemples, combinée à une répartition avantageuse des éléments génétiques rétroviraux, cette organisation du génome adénoviral permet d'obtenir une efficacité et une sécurité importantes. En outre, la structure génomique des adénovirus recombinants utilisés permet également la réalisation de répartitions des éléments génétiques rétroviraux originales et avantageuses, telles que notamment l'utilisation d'une combinaison de deux adénovirus tels qu'illustrés ci-avant. Les résultats montrent, de manière particulièrement avantageuse, que l'utilisation de ce type d'adénovirus et des répartitions particulières des éléments génétiques rétroviraux selon l'invention permettent d'obtenir des titres élevés de particules rétrovirales infectieuses et une stabilité à long terme du transgène (au-delà de 3 mois). Ces résultats tout à fait avantageux sont surprenants dans la mesure où l'absence de la région virale E4 a été décrite pour avoir un effet négatif sur l'expression des séquences introduites dans un vecteur adénoviral. Par ailleurs, les résultats présentés montrent que, in vivo, les compositions selon l'invention induisent une amplification d'un facteur 10 à 50 au moins du nombre de cellules tumorales transduites, par rapport à l'administration d'un adénovirus seul. Ces résultats confirment donc les propriétés avantageuses et surprenantes de la présente invention pour le transfert d'acides nucléiques dans les cellules.

Les adénovirus utilisés pour la mise en oeuvre de la présente invention sont donc

avantageusement d'adénovirus recombinants défectifs "dits" de 3ème génération, c'est-à-dire défectifs pour les régions E1 et E4, en tout ou en partie, et pour la région E3.

Des variantes particulières de l'invention sont constituées par l'utilisation d'adénovirus portant des délétions affectant tout ou une partie fonctionnelle des régions suivantes :
- E1, E4, E2 et E3,
- les régions ci-dessus ainsi que tout ou partie des gènes codant pour les fonctions tardives de l'adénovirus (L1 à L5), ou encore,
- l'ensemble des régions virales codantes.

La structure génomique des adénovirus a été largement décrite dans la littérature. A cet égard, le génome de l'adénovirus Ad5 a été entièrement séquencé et est accessible sur base de données (voir notamment GeneBandk M73260). De même, des parties, voire la totalité d'autres génomes adénoviraux (Ad2, Ad7, Ad12, adénovirus canin CAV-2, etc.) ont également été séquencées. De plus, la construction d'adénovirus recombinants défectifs a aussi été décrite dans la littérature. Ainsi, les demandes WO 94/28152, WO 95/02697 et WO 96/22378 par exemple, décrivent différentes délétions dans les régions E1 et E4. De même, la demande WO 96/10088 décrit des vecteurs portant une modification au niveau du gène Iva2, la demande WO 94/26914 décrit des adénovirus d'origine animale, la demande WO 95/29993 décrit des délétions portant sur la région E2 de l'adénovirus.

Avantageusement, l'adénovirus recombinant utilisé dans le cadre de l'invention comprend une délétion dans la région E1 de son génome affectant les régions E1a et E1b. A titre d'exemple précis, on peut citer des délétions affectant les nucléotides 454-3328; 382-3446 ou 357-4020 (par référence au génome de l'Ad5).

D'autre part, la délétion dans la région E4 affecte préférentiellement l'ensemble des phases ouvertes, telles que par exemple les délétions 33466-35535 ou 33093-35535 ou une partie seulement de la région E4 (ORF6 ou ORF3 par exemple), comme décrit dans les demandes WO95/02697 et WO96/22378.

Concernant les adénovirus dépourvus en plus des fonctions tardives (vecteur "minimum") ou de l'ensemble des régions codantes (vecteur "gutless"), leur construction a été décrite par exemple par Parks et al. PNAS 93 (1996) p. 13565 et Lieber et al., J. Virol. 70 (1996) p. 8944.

Les éléments génétiques rétroviraux peuvent être insérés en différents sites du génome adénoviral recombinant. Ils peuvent être insérés au niveau de la région E1. E3 ou E4, en remplacement des séquences délétées ou en surplus. Ils peuvent également être insérés en tout autre site, en dehors des séquences nécessaires en cis à la production des virus (séquences ITR et séquence d'encapsidation).

Par ailleurs, les adénovirus recombinants peuvent être d'origine humaine ou animale. Concernant les adénovirus d'origine humaine, on peut citer préférentiellement ceux classés dans le groupe C, en particulier les adénovirus de type 2 (Ad2), 5 (Ad5) ; ou les adénovirus 7 (Ad7) ou 12 (Ad12). Parmi les différents adénovirus d'origine animale, on peut citer préférentiellement les adénovirus d'origine canine, et notamment toutes les souches des adénovirus CAV2 [souche manhattan ou A26/61 (ATCC VR-800) par exemple]. D'autres adénovirus d'origine animale sont cités notamment dans la demande WO94/26914.

Les adénovirus recombinants sont produits dans une lignée d'encapsidation, c'est-à-dire une lignée de cellules capables de complémenter en trans une ou plusieurs des fonctions déficientes dans le génome adénoviral recombinant. Parmi les lignées d'encapsidation connues par l'homme du métier, on peut citer par exemple la lignée 293 dans laquelle une partie du génome de l'adénovirus a été intégrée. Plus précisément, la lignée 293 est une lignée de cellules embryonnaires humaines de rein contenant l'extrémité gauche (environ 11-12 %) du génome de l'adénovirus sérotype 5 (Ad5), comprenant l'ITR gauche, la région d'encapsidation, la région E1, incluant E1a et E1b, la région codant pour la protéine pIX et une partie de la région codant pour la protéine pIVa2. Cette lignée est capable de trans-complémenter des adénovirus recombinants défectifs pour la région E1, c'est-à-dire dépourvus de tout ou partie de la région E1, et de produire des stocks viraux ayant des titres élevés. Cette lignée est également capable de produire, à température permissive (32°C), des stocks de virus comportant en outre la mutation E2 thermosensible. D'autres lignées cellulaires capables de complémenter la région E1 ont été décrites, basées notamment sur des cellules de carcinome de poumon humain A549 (WO94/28152) ou sur des rétinoblastes humains (Hum. Gen. Ther. (1996) 215). Par ailleurs, des lignées capables de trans-complémenter plusieurs fonctions de l'adénovirus ont également été décrites. En particulier, on peut citer des lignées complémentant les régions E1 et E4 (Yeh et al., J. Virol. Vol. 70 (1996) pp 559-565; Cancer Gen. Ther. 2 (1995) 322 ; Krougliak et al., Hum. Gen. Ther. 6 (1995) 1575) et des lignées complémentant les régions E1 et E2 (WO94/28152, WO95/02697, WO95/27071) ou des lignées dérivées de celles-ci utilisables pour produire des adénovirus minimum, en particulier parce qu'elles expriment en outre une activité de recombinase site-spécifique impliquée dans la construction de tels virus.

Les adénovirus recombinants sont habituellement produits par introduction de l'ADN viral dans la lignée d'encapsidation, suivie d'une lyse des cellules après environ 2 ou 3 jours (la cinétique du cycle adénoviral étant de 24 à 36 heures). Pour la mise en oeuvre du procédé, l'ADN viral introduit peut être le génome viral recombinant complet, éventuellement construit dans une bactérie (WO96/25506) ou dans une levure (WO95/03400), transfecté dans les cellules. Il peut également s'agir d'un virus recombinant utilisé pour infecter la lignée d'encapsidation. L'ADN viral peut aussi être introduit sous forme de fragments portant chacun une partie du génome viral recombinant et une zone d'homologie permettant, après introduction dans la cellule d'encapsidation, de reconstituer le génome viral recombinant par recombinaison homologue entre les différents fragments.

Après la lyse des cellules, les particules virales recombinantes peuvent être isolées par toute technique connue telle que la centrifugation en gradient de chlorure de césium ou la chromatographie. Une méthode alternative a été notamment décrite dans la demande FR 9608164.

Les compositions selon l'invention peuvent comprendre des quantités variables d'adénovirus recombinants, aisément adaptables par l'homme du métier en fonction des applications envisagées (in vitro, ex vivo ou in vivo, par exemple).

Généralement, les compositions comprennent de l'ordre de 10⁵ à 10¹⁵ p.v. de chaque adénovirus recombinant, de préférence de 10⁷ à 10¹² p.v.

Le terme p.v. correspond au nombre de particules virales présentes dans les compositions.

Par ailleurs, les compositions de l'invention peuvent se présenter sous différentes formes, telles que solutions, gels, poudre, etc. Elles sont généralement sous forme de solutions, préférentiellement stériles, telles que par exemple des solutions salines (phosphate monosodique, disodique, chlorure de sodium, potassium, calcium ou magnésium, etc, ou des mélanges de tels sels), isotoniques, ou de compositions sèches, notamment lyophilisées, qui, par addition selon le cas d'eau stérilisée ou de sérum physiologique, permettent la constitution de solutés. D'autres excipients peuvent être utilisés tels que par exemple un hydrogel. Cet hydrogel peut être préparé à partir de tout polymère (homo ou hétéro) bio-compatible et non cytotoxique. De tels polymères ont par exemple été décrits dans la demande WO93/08845.

En outre, les compositions selon l'invention peuvent être conditionnées dans tout type de dispositif adapté, tel que flacon, tube, ampoule, poche, seringue, ballonet, etc. D'autre part, dans les compositions de l'invention comprenant au moins deux types d'adénovirus recombinants, ceux-ci peuvent être conditionnés soit en mélange, soit séparément.

Comme indiqué ci-avant, les compositions de l'invention peuvent être utilisées in vitro, ex vivo ou in vivo.

Pour une utilisation in vitro ou ex vivo, les cellules peuvent être simplement incubées, dans tout dispositif approprié (plaque, boîte, poche, etc.) en présence d'une composition telle que décrite ci-avant. Comme illustré sur la Figure 1 pour une composition comprenant deux types d'adénovirus recombinants, l'incubation des cellules entraîne l'infection des cellules par les deux adénovirus, qui permettent ensuite à la cellule co-infectée de produire des particules rétrovirales infectieuses. Pour ce type d'application, les compositions peuvent être utilisées à des multiplicités d'infection (MOI) comprises entre 10 et 5000 p.v. par cellule, par exemple, de préférence entre 100 et 2000, comme illustré notamment dans les exemples.

A cet égard, l'invention concerne également un procédé de production de particules rétrovirales in vitro comprenant l'incubation de cellules en présence d'une composition telle que définie ci-avant, éventuellement suivie de la récupération et/ou purification des rétrovirus produits. Les cellules utilisables dans ce procédé peuvent être tout type de cellule permissive aux adénovirus. Il peut s'agir de cultures primaires ou de lignées cellulaires, notamment mammifère, et notamment d'origine humaine. L'utilisation de cellules d'origine humaine (cellules de reins embryonnaires, A549, rétinoblastes, Hela, KB, etc.) est particulièrement avantageuse car elle permet de conférer une meilleure résistance des particules rétrovirales au système du complément. Comme illustré dans les exemples, le procédé de l'invention permet d'obtenir des titres élevés de rétrovirus, sans recours à des lignées d'encapsidation.

Pour une utilisation in vivo, les compositions de l'invention peuvent être formulées en vue d'une administration par voie topique, orale, parentérale, intranasale, intraveineuse, intramusculaire, sous-cutanée, intraoculaire, transdermique, intratumorale, etc.

A cet égard, l'invention concerne également l'utilisation d'une composition telle que décrite ci-avant pour le transfert d'un acide nucléique in vivo. L'invention peut être utilisée par exemple chez l'animal, pour la création de modèles pathologiques, ou l'étude de la régulation de gènes, ou également chez l'homme, dans des études de marquage, de biodisponibilité, ou à des fins médicales. L'invention concerne également l'utilisation des compositions décrites ci-avant pour la préparation d'un médicament destiné à la production de particules rétrovirales infectieuses in vivo.

Selon l'acide nucléique d'intérêt (transgène) inséré dans le vecteur rétroviral, la présente invention peut être utilisée dans de nombreuses applications. Ainsi, parmi les produits d'intérêt au sens de la présente invention, on peut citer plus particulièrement les enzymes, les dérivés sanguins, les hormones telles que l'hormone de croissance, les cytokines, les lymphokines : interleukines, interférons, TNF, etc. (Brevet français n° 92 03120), les facteurs de croissance, par exemples les facteurs angiogéniques tels que les VEGF ou FGF, les neurotransmetteurs ou leurs précurseurs ou enzymes de synthèse, les facteurs trophiques, en particulier neurotrophiques pour le traitement des maladies neurodégénératives, des traumatismes ayant endommagé le système nerveux, ou des dégénérescences rétiniennes : BDNF, CNTF, NGF, IGF, GMF aFGF, NT3, NT5, HARP/pléiotrophine, ou les facteurs de croissance osseuse, les facteurs hématopoïétiques, etc., la dystrophine ou une minidystrophine (brevet français n° 91 11947), les gènes codant pour des facteurs impliqués dans la coagulation : facteurs VII, VIII, IX, les gènes suicides (thymidine kinase, cytosine déaminase), les protéines impliquées dans le cycle cellulaire comme p21, ou autres protéines inhibitrices des kinases dépendantes, Rb, Gas-1, Gas-6, Gas-3, Gad 45, Gad 153, les cyclines A, B, D, ou encore la protéine GAX limitant la prolifération des cellules dans les muscles lisses (traitement de la resténose), les protéines induisant l'apoptose ou d'autres suppresseurs de tumeurs comme p53, Bax, BclX-s, Bad ou tout autre antagoniste de Bcl2 et de BclX-1, les gènes de l'hémoglobine ou d'autres transporteurs protéiques, les gènes correspondant aux protéines impliquées dans le métabolisme des lipides, de type apolipoprotéine choisie parmi les apolipoprotéines A-I, A-II, A-IV, B, C-I, C-II, C-III, D, E, F, G, H, J et apo(a), les enzymes du métabolisme comme par exemple la lipoprotéine lipase, la lipase hépatique, la lécithine cholestérol acyltransférase, la 7 alpha cholestérol hydroxylase, la phosphatidyl acide phosphatase, ou encore des protéines de transfert de lipides comme la protéine de transfert des esters de cholestérol et la protéine de transfert des phospholipides, une protéine de liaison des HDL ou encore un récepteur choisi parmi les récepteurs aux LDL, les récepteurs des chylomicrons-remnants et les récepteurs scavenger, etc.

Parmi les produits d'intérêt il est important de souligner les anticorps, les fragments variables d'anticorps simple chaîne (ScFv) ou tout autre fragment d'anticorps possédant des capacités de reconnaissance pour son utilisation en immunothérapie, par exemple pour le traitement des maladies infectieuses, des tumeurs (anticorps anti-RAS, anti-p53 ou anti-GAP), des maladies autoimmunes telles que la sclérose en plaques (anticorps antiidiotype).

D'autres protéines d'intérêt sont, de façon non limitative, des récepteurs solubles, comme par exemple le récepteur CD4 soluble ou le récepteur soluble du TNF pour la thérapie anti-HIV, le récepteur soluble de l'acétylcholine pour le traitement de la myasthénie ; des peptides substrats ou inhibiteurs d'enzymes, ou bien des peptides agonistes ou antagonistes de récepteurs ou de protéines d'adhésion comme par exemple pour le traitement de l'asthme, de la thrombose et de la resténose : des protéines artificielles, chimériques ou tronquées. Parmi les hormones d'intérêt essentiel, on peut citer l'insuline dans le cas du diabète, l'hormone de croissance et la calcitonine.

L'acide nucléique peut également être un gène ou une séquence antisens, dont l'expression dans la cellule cible permet de contrôler l'expression de gènes ou la transcription d'ARNm cellulaires. De telles séquences peuvent, par exemple, être transcrites dans la cellule cible en ARN complémentaire d'ARNm cellulaires et bloquer ainsi leur traduction en protéine, selon la technique décrite dans le brevet européen n° 140 308. Les gènes thérapeutiques comprennent également les séquences codant pour des ribozymes, qui sont capables de détruire sélectivement des ARN cibles (brevet européen n° 321 201).

L'acide nucléique peut également comporter un ou plusieurs gènes codant pour un peptide antigénique, capable de générer chez l'homme ou l'animal une réponse immunitaire. Dans ce mode particulier de mise en oeuvre, l'invention permet donc la réalisation soit de vaccins, soit de traitements immunothérapeutiques appliqués à l'homme ou à l'animal, notamment contre des microorganismes, des virus ou des cancers. Il peut s'agir notamment de peptides antigèniques spécifiques du virus d'Epstein Barr, du virus HIV, du virus de l'hépatite B (brevet Européen N° 185 573), du virus de la pseudo-rage, du "syncitia forming virus", d'autres virus ou encore d'antigènes spécifiques de tumeurs comme les protéines MAGE (brevet Européen n° 259 212).

L'acide nucléique peut également coder pour un produit toxique pour les cellules, notamment une toxicité conditionnelle (i.e., thymidine kinase, cytosine désaminase, etc.).

D'autres gènes présentant un intérêt ont été notamment décrits par Mc Kusick, V.A. Mendelian (Inheritance in man, catalogs of autosomal dominant, autosomal recessive, and X-linked phenotypes, Eighth edition. John Hopkins University Press (1988)), et dans Standbury, J. B. et al. (The metabolic basis of inherited disease, Fifth edition. McGraw-Hill (1983)). Les gènes d'intérêt recouvrent les protéines impliquées dans le métabolisme des acides aminés, des lipides et des autres constituants de la cellule.

Enfin, l'acide nucléique peut comporter plusieurs régions codantes, éventuellement séparées par une IRES, permettant la production de plusieurs produits d'intérêt.

Par ailleurs, le transgène comporte généralement un promoteur transcriptionnel (situé en 5') et un terminateur transcriptionnel (situé en 3'), qui peuvent être choisis par l'homme du métier, comme décrit ci-avant. D'autre part, le transgène peut être présent dans le vecteur rétroviral dans l'orientation identique ou opposée au sens de la transcription des LTR. Enfin, dans un mode particulier de mise en oeuvre, les génomes adénoviraux de l'invention peuvent également être délivrées aux cellules sous forme plasmidique, en combinaison avec les fonctions de complémentation adénovirales.

### LÉGENDES DES FIGURES

TABLEAU 1 : Mesure de l'activité RT dans les cellules W162 infectées ou co-infectées, aux MOI indiquées, avec AdTK/ENV et/ou AdGAG/POL. L'activité RT a été mesurée 2 jours après l'infection à partir des surnageants, comme décrit dans l'exemple 5.2. L'activité RT de cellules GP +envAM12 à servi de comparaison. Les résultats présentés sont la moyenne de 2 expériences indépendantes au moins (Ecart type inférieur à 10%).

FIGURE 1 : Schéma général de l'utilisation d'une composition selon l'invention comprenant deux vecteurs chimériques rétro/adénoviraux = Adétrovirus.

FIGURE 2 : Représentation des cassettes d'expression des fonctions de complémentation rétrovirales (2B, 2C) et du vecteur rétroviral (2A).

FIGURE 3 : Représentation du plasmide navette pAdEGP. E./P. EF-1α : Enhancer / Promoteur du gène EF-1α; pA : site de polyadénylation.

FIGURE 4 : Représentation du plasmide navette pAd CLTKEE. E./P. CMV: Enhancer/Promoter CMV ; pA: site de polyadénylation.

FIGURE 5 : Mécanisme de production de particules rétrovirales infectieuses au moyen d'une composition de l'invention.

FIGURE 6 : Cinétique de production de particules rétrovirales après co-infection de cellules W162 avec Ad TK/ENV et Ad GAG/POL.

FIGURE 7 : Production de particules rétrovirales dans les cellules W162, en fonction de la MOI de l'infection adénovirale.

FIGURE 8 : Quantification par Slot-Blot des ARNs TK+ encapsidés dans les particules virales.

FIGURE 9 : Schéma de détection, par PCR et coupure enzymatique, de la présence d'une cassette rétrovirale TK intégrée.

FIGURE 10 : Schéma de détection, par PCR et coupure enzymatique, de la présence d'une cassette rétrovirale TK intégrée ou non intégrée.

FIGURE 11 : Mise en évidence de la sensibilité au gancyclovir des cellules infectées par les rétrovirus produits, comprenant le gène TK.

FIGURE 12 : Amplification du gène TK dans les tumeurs H460 in vivo. Ligne M : marqueur 1 kb (Gibco BRL) ; ligne - : pas d'ADN ; ligne + : plasmide pLTK.

FIGURE 13 : Amplification du provirus TK dans les tumeurs H460 in vivo.

### Techniques générales de biologie moléculaire

Les méthodes classiquement utilisées en biologie moléculaire telles que les extractions préparatives d'ADN plasmidique, la centrifugation d'ADN plasmidique en gradient de chlorure de césium, l'électrophorèse sur gels d'agarose, la purification de fragments d'ADN, les extractions de protéines au phénol-chloroforme, la précipitation d'ADN en milieu salin par de l'éthanol ou de l'isopropanol, la transformation dans *Escherichia coli,* etc ... sont bien connues de l'homme de métier et son abondamment décrites dans la littérature [Maniatis T. et al., "Molecular Cloning, a Laboratory Manual", Cold Spring Harbor Laboratory, Cold Spring Harbor, N.Y., 1982; Ausubel F.M. et al. (eds), "Current Protocols in Molecular Biology", John Wiley & Sons, N.Y. 1987].

Pour les ligatures, les fragments d'ADN peuvent être séparés selon leur taille par électrophorèse en gels d'agarose, purifiés en utilisant le kit "QlAquick Gel Extraction Kit" distribué par Qiagen puis incubés en présence de l'ADN ligase du phage T4 (Biolabs) selon les recommandations du fournisseur. La déphosphorylation des extrémités 5' de l'ADN peut être effectuée par la phosphatase alkaline (Boeringer Mannheim) selon les spécifications du fournisseur. Le remplissage des extrémités 5' proéminentes peut être effectué par le fragment de Klenow de l'ADN polymérase 1 d'*E*. *coli* (Biolabs) selon les recommandations du fabricant.

La mutagénèse dirigée *in vitro* par oligodéoxynucléotides synthétiques peut être effectuée selon la méthode développée par Mikaelian and Sergeant [Nucleic Acids Research, 1992, 20: 376]. L'amplification de fragments d'ADN par la technique dite de PCR [Polymerase-catalysed Chain Reaction, Saiki R.K. et al., 1985, Science 230: 1350-1354] peut être effectuée en utilisant l'ADN polymérase *Pfu* (Stratagène) selon les spécifications du fabricant. La vérification des séquences nucléotidiques peut être effectuée par la méthode développée par Sanger et al. [Proc. Natl. Acad. Sci. USA, 1977, 74: 5463-5467].

Les cellules IGRP2 et W162 (Weinberg et al., PNAS 80 (1983) 5383) ont été cultivées en milieu minimum Eagle (MEM) supplémenté de 10% sérum bovin foetal (SBF). Les cellules de la lignée NCI-H460 de carcinome pulmonaire non à petites cellules, d'origine humaine (ATCC-HTB 177) et les fibroblastes murins NIH-3T3 ont été cultivés en milieu Dulbecco modifié (DMEM) et RPMI 1640, respectivement, supplémenté de 10% SBF. Toutes les cellules ont été cultivées à 37°C dans 5% CO₂.

### Exemple 1. Construction de 3 plasmides portant différentes unités rétrovirales sous contrôle d'un promoteur.

Cet exemple décrit la construction de 3 plasmides contenant, respectivement, les cassettes d'expression (i) de l'enveloppe rétrovirale 4070A, (ii) des protéines gag-pol du virus MoMLV et (iii) du gène *tk* d'HSV-1 placé dans un contexte rétroviral.

### 1.1. Construction du plasmide pEF1a-Env

Le fragment Hind3/Xba1 contenant le promoteur/enhancer du gène EF-1a issu du plasmide pEF-BOS [Mizushima and Nagata, 1990, Nucl. Acids Res. 18: 5322] est cloné dans les sites Hind3 et Xba1 du plasmide commercial pSI (Promega) pour générer le plasmide pSl-EF1a.

Un fragment Xba1/Not1 contenant le gène de l'enveloppe du virus MLV amphotrope 4070A (*env* 4070A) est amplifié par PCR en utilisant le plasmide pSV-envAM (don de T. Heidmann, CNRS URA 147) comme matrice et les oligonucléotides SEQ ID N°1 et SEQ ID N°2 décrits ci-dessous.
SEQ ID N°1
   5'- GGC*TCTAGA***GCCGCCACCATGG**CGCGTTCAACGCTC-3'

Le site Xba1 est en italique, la séquence consensus Kozak d'initiation de la traduction est en gras.
SEQ ID N°2
   5'-AAAAAAAA*GCGGCCGC***TTATCA**TGGCTCGTACTCTATGG-3'

Le site Not1 est en italique, les deux codons Stop sont en gras.

Au cours de cette amplification, une réaction PCR intermédiaire avec les oligonucléotides SEQ ID N°3 et SEQ ID N°4 a permis d'éliminer le site Pac1 présent dans le gène env sans modifier la séquence d'acides aminés. SEQ ID N°3
5'-CAGGCATGGAGTCAAAACCAGAGCCTGGAC-3'
SEQ ID N°4
   5'-CACTGCC**TTAATCAA**AACCCAGCAG-3'

Le site Pac1 modifié est en gras.

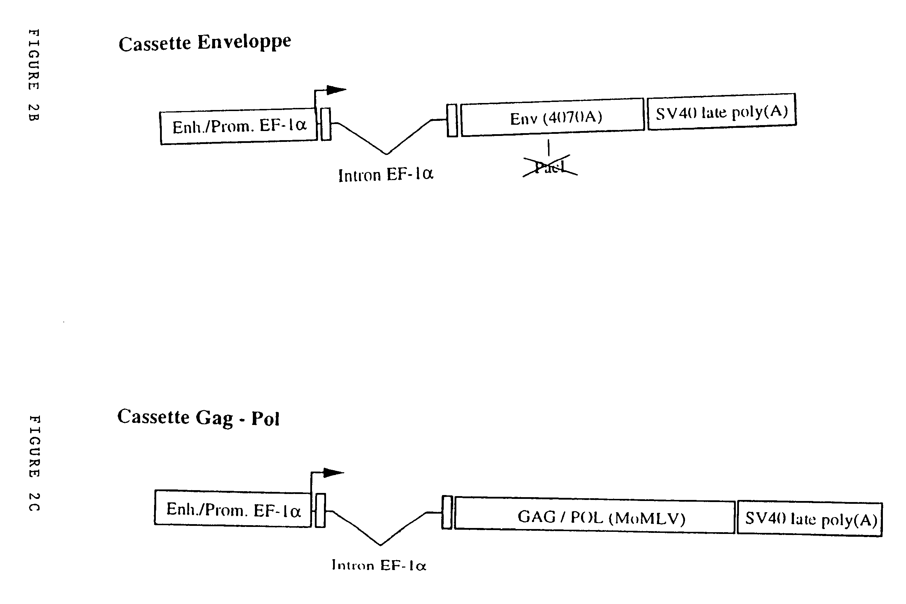

Le fragment Xba1/Not1 amplifié, contenant le gène *env* 4070A, est cloné dans les sites Xba1 et Not1 de pSI-EF1a pour générer le plasmide pEF1 a-Env qui contient donc le gène *env*4070A placé sous le contrôle du promoteur EF-1 a et suivi du signal de polyadénylation tardif du virus SV40. La cassette Env portée par ce plasmide est représentée sur la Figure 2B.

### 1.2. Construction du plasmide pEF1a-GP+

Le fragment Xma1/Xma1 de pMOV-3ACla (don de T. Heidmann, CNRS URA 147) contenant le cistron *gag* et une partie du cistron *pol* du virus MLV de souche Moloney est cloné dans le site Xma1 de pSl-EF1a (voir paragraphe 1.1.) pour générer le plasmide pEF1a-GP.

Un fragment EcoR1/BsrG1 contenant la partie 5' du cistron *gag* est amplifié par PCR en utilisant le plasmide pMOV-3ΔCla comme matrice avec les oligonucléotides SEQ ID N°5 et SEQ ID N°6 décrits ci-dessous.
SEQ ID N°5
   5'-CCG*GAATTC***GCCGCCACCATGG**GCCAGACTGTTACC-3'

Le site EcoR1 est en italique, la séquence consensus Kozak d'initiation de la traduction est en gras.
SEQ ID N°6
   5'-GGAGGCGGAGGCTTAGGGTG-3'.

Le fragment EcoR1/BsrG1 amplifié est cloné dans les sites EcoR1 et BsrG1 de pEF1a-GP pour générer le plasmide pEF1a-GPaug.

Un fragment Xma1/Not1 contenant la partie 3' du cistron *pol* est amplifié par PCR en utilisant le plasmide pMOV-3ΔCla comme matrice avec les oligonucléotides SEQ ID N°7 et SEQ ID N°8 décrits ci-dessous.
SEQ ID N°7
   5'-GTAGACGGCATCGCAGCTTG-3'
SEQ ID N°8
   5'-AAAAAAAA*GCGGCCGC***TCATTA**GGGGGCCTCGCGGG-3'

Le site Not1 est en italique, les codons Stop sont en gras.

Le fragment Xma1/Not1 amplifié est cloné dans les sites Xma1 et Not1 de pEF1a-GPaug et génère le plasmide pEF1a-GP+ qui contient donc le cistron *gag-pol* placé sous le contrôle du promoteur EF-1 a et suivi du signal de polyadénylation tardif du virus SV40. La cassette GAG/POL portée par ce plasmide est représentée sur la Figure 2C.

### 1.3. Construction du plasmide pCLTKRpA

Le fragment BstY1/Nar1 de pXL3022, contenant le gène *tk* d'HSV-1 et son leader, est cloné dans les sites Bcl1 et Cla1 (sites compatibles) du vecteur rétroviral pLNCX [Miller and Rosman, 1989, Biotechniques 7: 980-990] pour générer pLTK.

Les fragments Sac1/Sac1 et Sac1/Nhe1 de pLTK sont clonés dans les sites Sac1 et Nhe1 du plasmide commercial pCI (Promega), contenant le promoteur/enhancer hCMV-IE (-733/-14), pour générer pCLTK.

Un fragment Nhe1/Not1 contenant les 3/4 de la séquence U3 et la séquence R, est amplifié par PCR en utilisant le plasmide pLNCX comme matrice avec les oligonucléotides SEQ ID N°9 et SEQ ID N°10 décrits ci-dessous :
SEQ ID N°9
   5'-GACCCCACCTGTAGGTTTGGC-3'
SEQ ID N° 10
   5'-AAAAAAAAGCGGCCGCTGCAACTGCAAGAGGG-3'

Le site Not1 est en italique. Le fragment Nhe1/Not1 amplifié est cloné dans les sites Nhe1 et Not1 de pCLTK pour générer pCLTKR.

Un fragment Not1/BamH1 contenant le signal de polyadénylation du gène *bgh* (bovine growth hormone) est amplifié par PCR en utilisant le plasmide commercial pcDNA3 (Invitrogen) comme matrice avec les oligonucléotides SEQ ID N°11 et SEQ ID N°12 décrits ci-dessous.
SEQ ID N°11
   5'-AAAAAAAAGCGGCCGCTAAATGCTAGAGCTCGCTG-3'

Le site Not1 est indiqué en italique
SEQ ID N°12
   5'-CGC*GGATC*CCCACCGCATCCCCAGC-3'

Le site BamH1 est indiqué en italique.

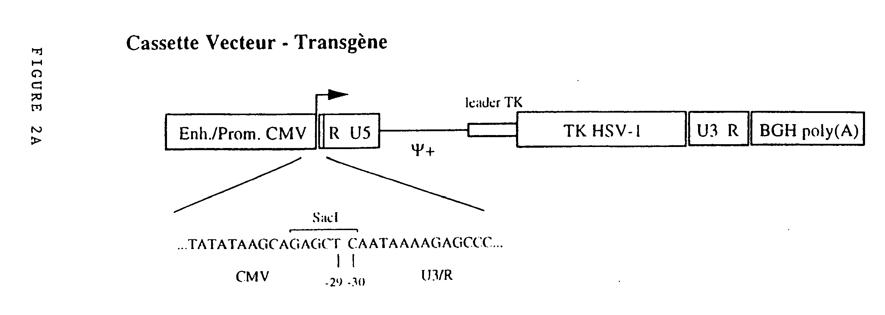

Le fragment Not1/BamH1 amplifié est cloné dans les sites Not1 et BamH1 de pCLTKR et génère pCLTKRpA qui contient donc le gène *tk* dans un contexte de vecteur rétroviral. Dans le LTR 5', la séquence U3 est remplacée par le promoteur/enhancer hCMV-IE. Il est décrit que ce LTR hybride (hCMV-R-U5) est fonctionnel [Naviaux et al., 1996, J. Virol. 70: 5701-5705]. Dans le LTR 3', la séquence U5 a été éliminée et remplacée par le signal de polyadénylation du gène *bgh.* La structure de la cassette obtenue est représentée sur la Figure 2A.

### Exemple 2. Construction de deux plasmides "navettes" portant différentes unités rétrovirales en vue de la technologie E. coli

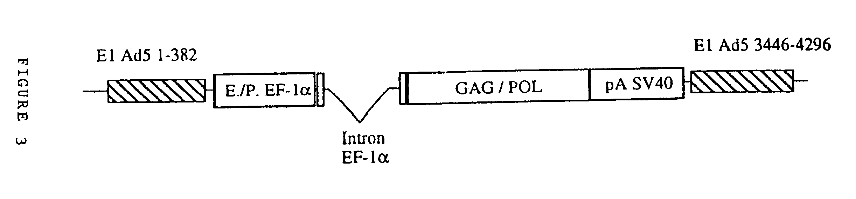

Cet exemple décrit la construction de deux plasmides dits "navettes" qui seront utilisés pour la construction de deux adénovirus recombinants par la technologie *E.coli* (voir exemple 3). Ils contiennent les cassettes d'expression TK et Env (pAdCLTKEE), et Gag-Pol (pAdEGP). (Figure 3)

### 2.1. Construction du plasmide "navette" pAdEGP (Figure 3)

Le fragment Fsp1/Fsp1 de pEF1a-GP+, contenant le cistron *gag-pol* sous le contrôle du promoteur EF-1a (voir paragraphe 1.2.), est cloné dans le site EcoRV déphosphorylé de pXL3048 (plasmide porteur des gènes de sélection *Kana* et *SacB* pour générer le plasmide pAdEGP qui contient ainsi les séquences 1-382 et 3446-4296 de l'adénovirus de type 5 respectivement en 5' et 3' de la cassette d'expression Gag-Pol.

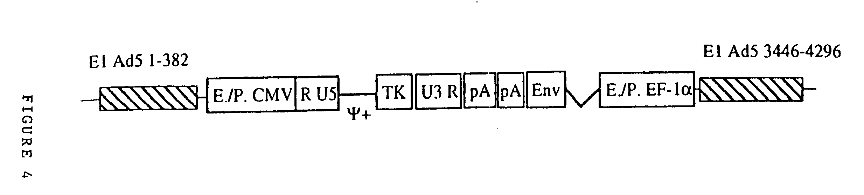

### 2.2. Construction du plasmide "navette" pAdCLTKEE (Figure 4)

Le fragment Bgl2/BamH1 de pCLTKRpA contenant la cassette TK dans un contexte rétroviral (voir paragraphe 1.3.) est cloné dans le site BamH1 de pXL3048 (plasmide porteur des gènes de sélection *Kana* et *SacB*; RPR-Gencell) pour générer le plasmide pAdCLTK.

Le fragment Avr2/Ear1 (traité Klenow) de pEF1a-Env contenant la cassette d'expression Env (voir paragraphe 1.1.) est cloné dans le site Sal1 (traité par la phosphatase alcaline puis Klenow) de pAdCLTK pour générer le plasmide pAdCLTKEE qui contient ainsi les séquences 1-382 et 3446-4296 de l'adénovirus de type 5 respectivement en 5' et 3' de la cassette d'expression TK-Env. La cassette d'expression Env4070A, localisée en 3' de la cassette d'expression TK, est transcrite dans le sens opposé.

### Exemple 3. Construction de 2 vecteurs adÈnoviraux recombinants E1⁻/E3⁻/E4⁻ portant différentes unités rétrovirales

Cet exemple décrit la construction de deux vecteurs adénoviraux recombinants E1⁻/E3⁻/E4⁻ contenant respectivement les cassettes d'expression TK-Env (AdTK/ENV) et Gag-Pol (AdGAG/POL) par la technologie de double recombinaison dans *E. coli.*

### 3.1. Technologie E.coli

La technologie *E. coli* permet la construction d'un génome adénoviral recombinant infectieux à partir d'un génome d'adénovirus 5 modifié et d'un plasmide "navette" (contenant la cassette à intégrer dans le génome adénoviral), après deux recombinaisons homologues successives dans une souche mutante *polA* d'*E*. *coli.* La technique utilisée a été largement décrite par Crouzet et al. [1997, Proc. Natl. Acad. Sci. USA 94: 1414-1419].

### 3.2. Construction des génomes recombinants AdGAG/POL et AdTK/ENV

Le génome adénoviral de type 5, utilisé pour la technologie *E*. *coli,* est cloné dans le plasmide pXL2811 (Crouzet et al. précité). Ce génome, localisé dans un fragment Pac1, présente des délétions dans la région E1 (ΔE1: délétion Hinfl-Bgl2 382-3328), E3 (ΔE3: délétion Xba1 28592-30470) et E4 (ΔE4: délétion Ssp1-Sma1 33423-35356).

Le génome adénoviral recombinant AdGAG/POL est construit après recombinaisons homologues dans *E. coli* entre le plasmide "navette" pAdEGP (voir paragraphe 2.1.) et pXL2811. La cassette d'expression Gag-Pol est localisée dans la délétion E1.

Le génome adénoviral recombinant AdTK/ENV est construit après recombinaisons homologues dans *E. coli* entre le plasmide "navette" pAdCLTKEE (voir paragraphe 2.2.) et pXL2811. La cassette d'expression TK-Env est localisée dans la délétion E1.

Les 2 préparations d'ADNs recombinants sont analysées par digestions enzymatiques puis électrophorèse sur gel d'agarose 0,7%. Elles sont également séquencées (sites de clonages, séquences amplifiées par PCR...). Le choix des enzymes de restriction permet d'étudier en particulier l'intégrité des trois cassettes d'expression TK, Env et Gag-Pol dans leur contexte adénoviral.

Le nombre de bandes obtenues, la taille de ces bandes et les régions séquencées correspondent totalement aux profils attendus. Aucune délétion ni modification ne sont observées dans les 2 génomes. Ces résultats indiquent d'une part que les 2 génomes adénoviraux construits sont corrects et d'autre part que les séquences rétrovirales intégrées, dont les LTRs modifiées, ne génèrent pas de recombinaisons internes lors de la construction dans *E. coli* assurant ainsi la stabilité génétique de l'ADN adénoviral.

### Exemple 4. Production et caractérisation des 2 préstocks viraux AdGAG/POL et AdTK/ENV

Cet exemple décrit la production, la caractérisation et la titration des deux adénovirus recombinants AdTK/ENV et AdGAG/POL.

### 4.1. Production des adénovirus AdTK/ENV et AdGAG/POL

Les ADNs AdTK/ENV et AdGAG/POL (20 µg) sont digérés par Pac1, afin de libérer le fragment Pac1 contenant strictement le génome adénoviral recombinant, puis sont précipités par de l'éthanol. Chacun des deux ADNs est transfecté avec de la LipofectAMINE (GibcoBRL), selon les indications du fournisseur, dans une lignée cellulaire 293 *trans*-complémentantes pour les protéines adénovirales E1 et E4 [cellules IGRP2; Yeh et al., 1996, J. Virol. 70: 559-565].

Les cellules sont maintenues en culture jusqu'à l'apparition d'un effet cytopathique (cellules décollées). A cette date, le surnageant est prélevé puis congelé/décongelé 3 fois avant d'être utilisé pour infecter de nouvelles cellules IGRP2. Ainsi, les deux adénovirus recombinants sont amplifiés successivement en plaque de culture 12 puits (ampli. 1), plaque de culture 6 puits (ampli. 2) et boites de culture de 10 cm de diamètre (ampli. 3 et 4). Pour chacun des virus, le préstock final d'environ 150 ml est prélevé sur 35 boites de 10 cm (ampli. 4). Il est également congelé/décongelé 3 fois puis conservé à -20°C.

### 4.2. Caractérisation de la stabilité génétique des virus AdTK/ENV et AdGAG/POL

II s'agit de vérifier l'intégrité des génomes adénoviraux AdTK/ENV et AdGAG/POL après l'amplification des virus en culture de cellules.

Un aliquot (2 ml) de chacun des deux préstocks est utilisé pour purifier l'ADN viral selon la méthode de Gluzman and Van Doren (1983, J. Virol. 45: 91-103). L'ADN purifié est analysé par digestions enzymatiques puis électrophorèse sur gel d'agarose 0,7%. Le choix des enzymes de restriction permet d'étudier en particulier l'intégrité des trois cassettes d'expression TK, Env et Gag-Pol dans leur contexte adénoviral.

Le nombre de bandes obtenues et la taille de ces bandes correspondent totalement aux profils attendus. Aucune délétion n'est observée dans les 2 génomes. Ces résultats indiquent que les séquences rétrovirales intégrées dans les deux génomes adénoviraux n'altèrent pas la stabilité génétique de ces génomes.

### 4.3. Titration des préstocks adénoviraux AdTK/ENV et AdGAG/POL

Les deux préstocks viraux obtenus ont été titrés selon deux méthodes: (i) immuno-titration avec un anticorps monoclonal anti-penton et (ii) High-Pressure Liquid Chomatography ou HPLC (F. Blanche, RPR-Gencell ), afin d'estimer en particulier la productivité de chacun des virus.

Pour l'immuno-titration, des cellules IGRP2 cultivées en plaque de 6 puits sont infectées avec des dilutions du préstock viral. Quarante huit heures après l'infection, les cellules sont fixées avec du méthanol 90% puis incubées 1 heure en présence de PBS/5% SBF (sérum bovin foetal) pour saturation. Les cellules sont ensuite incubées avec un anticorps monoclonal anti-penton (Biodesign International) dilué au 1/100, lavées puis marquées avec un anticorps lié à la peroxydase dilué au 1/500 (Amersham). Les cellules marquées sont révélées en présence de 3,3'-Diaminobenzidine (DAB)/H₂O₂.

Le nombre de particules infectieuses (PI) évalué par immuno-titration est de 5,8 x 10⁸ PI/ml pour AdTK/ENV et de 3,3 x 10⁸ PI/ml pour AdGAG/POL. Le nombre de particules virales (PV) déterminé par HPLC est de 1,9 x 10¹⁰ PV/ml pour AdTK/ENV et de 1,3 x 10¹⁰ PV/ml pour AdGAG/POL. Le rapport PV/PI est de 33 (AdTK/ENV) et de 39 (AdGAG/POL).

Les titres viraux obtenus démontrent que les deux préstocks AdGAG/POL et AdTK/ENV ont une bonne productivité. Celle-ci est comparable à la productivité d'autres adénovirus recombinants défectifs pour E1 et E4. Ces données impliquent, en particulier, que la présence de séquences rétrovirales et de leurs fonctions n'interfèrent pas avec les séquences adénovirales et leurs fonctions dans la productivité des virus.

### Exemple 5. Fonctionnalité des cassettes d'expression rétrovirales présentes dans les adénovirus AdTK/ENV et AdGAG/POL

Cet exemple décrit l'étude de la fonctionnalité de la cassette d'expression TK présente dans l'adénovirus AdTK/ENV et de la cassette d'expression Gag-Pol présente dans AdGAG/POL. La fonctionnalité de la cassette d'expression Env sera testée par l'intermédiaire de la titration des rétrovirus recombinants produits (voir paragraphe 6.4.).

### 5.1. Fonctionnalité de la cassette d'expression TK

La fonctionnalité de la cassette d'expression est analysée de 3 manières: (i) quantification des ARNs TK⁺, (ii) mise en évidence de la protéine TK dans le lysat cellulaire et (iii) marquage fluorimétrique des cellules exprimant la protéine TK. Des cellules infectées avec AdGAG/POL et un adénovirus recombinant exprimant le gène *tk* constituent, respectivement, un témoin négatif et un témoin positif pour chacun des essais.

Quarante huit heures après l'infection de cellules W162 (cellules dérivées de cellules Vero, issues du rein de singe vert) avec AdTK/ENV (moi 10 à 837 PI), les ARNs totaux sont extraits avec le kit RNeasy (Qiagen) selon les indications du fournisseur. Les ARNs (15 µg) sont déposés sur une membrane nylon puis hybridés avec une sonde TK radioactive selon les protocoles décrits par Maniatis T. et al. ["Molecular Cloning, a Laboratory Manual", Cold Spring Harbor Laboratory, Cold Spring Harbor, N.Y., 1982]. Cette analyse permet de quantifier spécifiquement les ARNs TK⁺.

Des cellules W162 infectées avec AdTK/ENV sont récoltées 48 heures p.i. puis lysées afin de révéler par test ELISA la présence de la protéine TK.

Des cellules W162 infectées avec AdTK/ENV (moi 50 à 200 PI) sont récoltées 48 heures p.i. fixées 30 min avec du formaldehyde 4% puis lysées 10 min avec du triton 0,2%. Les cellules sont ensuite incubées avec un anticorps IgG lapin anti-TK (pAbTK41, RPR-Gencell) dilué au 1/250, lavées (tampon Power-Block 1 X, Biogenex) puis marquées avec un anticorps anti-IgG lapin couplé FITC dilué au 1/250 (Jackson ImmunoResearch Laboratories). Les cellules marquées sont analysées par cytométrie de flux (FACS).

Les résultats indiquent que des ARNs TK⁺ sont transcrits. La quantité de ces ARNs TK⁺ transcrits est proportionnelle à la moi de l'infection adénovirale AdTK/ENV. La protéine TK est détectée dans le lysat de cellules infectées et 70 à 90 % des cellules infectées (selon la moi) sont marquées positivement.

L'ensemble de ces données indiquent clairement que la cassette d'expression TK est fonctionnelle tant sur le plan transcriptionnel que traductionnel.

### 5.2. Fonctionnalité de la cassette d'expression Gag-Pol

La fonctionnalité de la cassette d'expression est étudiée en recherchant la présence de l'activité reverse-transcriptase, codée par le cistron *pol*, dans le surnageant de cellules W162 infectées avec AdGAG/POL. Dans le surnageant de culture, l'enzyme reverse-transcriptase (RT) est présente dans la particule rétrovirale dont les constituants sont codés par le cistron *gag*. La présence d'une activité reverse-transcriptase démontre donc la présence de particules rétrovirales c'est à dire la présence des protéines gag. Des surnageants de cellules W162 infectées avec AdTK/ENV et de cellules GP+*env*AM12 (lignée murine productrice de particules rétrovirales; [Markowitz et al., 1988, Virology 167: 400-406]) constituent, respectivement, un témoin négatif et un témoin positif pour l'essai.

Un aliquot (30 µl) de surnageant de cellules W162 infectées avec AdGAG/POL (moi 10 à 500 PI) est prélevé 48, 72 et 96 heures p.i. et utilisé dans un test reverse-transcriptase (test RT) selon le protocole décrit par Goff et al. [1981, J. Virol. 38: 239-248].

Le test RT révèle la présence d'une activité reverse-transcriptase, donc de particules rétrovirales, dans le surnageant de cellules infectées par AdGAG/POL. La production de particules rétrovirales est maximale pour une moi d'environ 200 PI (résultats de 2 essais indépendants).

Ces données indiquent clairement que la cassette d'expression Gag-Pol est fonctionnelle.

### Exemple 6. Production de particules rétrovirales recombinantes in vitro après co-infection adénovirale AdTK/ENV-AdGAG/POL

Cet exemple décrit l'utilisation de cellules infectées simultanément (co-infection) avec AdTK/ENV et AdGAG/POL pour la production de particules rétrovirales recombinantes infectieuses *in vitro.* Le schéma général de production des particules virales est présenté sur la Figure 5.

### 6.1. Co-infection adénovirale AdTK/ENV-AdGAG/POL

II s'agit de comparer l'activité reverse-transcriptase, c'est à dire la productivité virale, de surnageants de cellules W162 co-infectées avec AdGAG/POL et AdTK/ENV (moi 5/5 PI) et de cellules W162 infectées avec AdGAG/POL seul (moi 5 PI). Des surnageants de cellules W162 infectées avec AdTK/ENV et de cellules GP+envAM12 constituent, respectivement, un témoin négatif et un témoin positif pour l'essai.

Un aliquot (30 µl) de chaque surnageant est prélevé 48, 72 et 96 heures post-infection puis utilisé pour un test RT.

Aucune différence de productivité n'est observée au cours du temps entre les cellules co-infectées et les cellules infectées avec AdGAG/POL seul démontrant qu'il n'y a aucune interférence entre les fonctions rétrovirales Gag-Pol et Env d'une part, et entre les fonctions adénovirales et rétrovirales d'autre part.

### 6.2. Cinétique de production des particules rétrovirales

II s'agit d'étudier la cinétique de production de particules rétrovirales après co-infection de cellules W162 avec AdTK/ENV et AdGAG/POL (moi 5/5 PI).

Un aliquot (30 µl) de surnageant est prélevé régulièrement pendant 15 jours post-infection puis utilisé pour un test RT. Le milieu de culture est changé 24 heures avant chaque prélèvement. Les cellules infectées sont maintenues en culture pendant 15 jours sans passages.

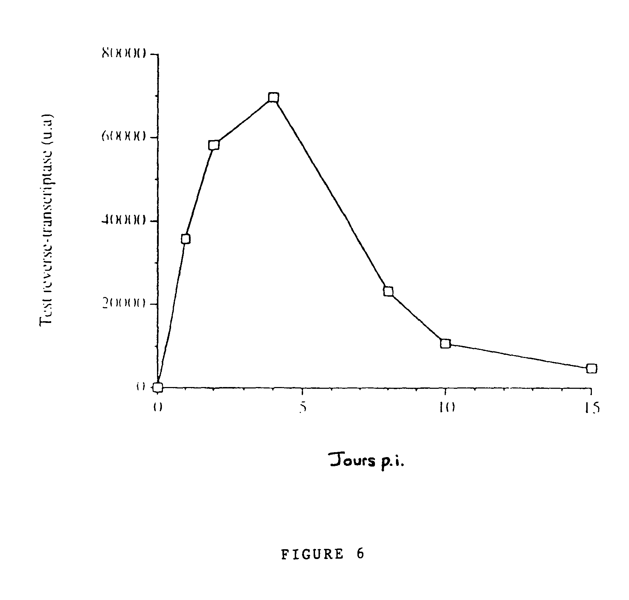

Les résultats obtenus sont présentés sur la Figure 6. Ils montrent que la production de particules rétrovirales augmente très rapidement entre 0 et 4 jours puis diminue progressivement entre 5 et 15 jours. Pendant les 7 premiers jours, le taux de production virale se situe entre 50 et 100%. L'apparition de plusieurs couches cellulaires après 1 semaine de culture peut amener à sous estimer la productivité virale: les virus transmis de cellules à cellules par fusion membranaire, sans être relargués dans le surnageant, ne sont pas détectés lors du test RT.

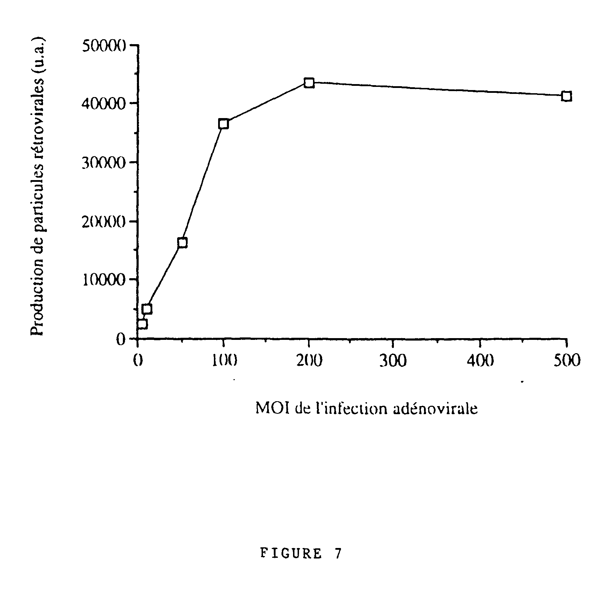

La Figure 7 et le Tableau 1 montrent la production de particules rétrovirales dans les cellules W162, en fonction de la MOI de l'infection avec les adénovirus Ad TK/ENV et Ad GAG/POL. Comme indiqué dans le Tableau 1, les cellules W162 co-infectées présentent une activité grossièrement proportionnelle aux MOI utilisées, jusqu'à 200 PI/cellule. A des MOI supérieures (300 et 350 PI/cellule), il n'y a pas d'augmentation supplémentaire de l'activité RT. La MOI de 200 semble donc optimale pour la production de particules rétrovirales. A cet égard, les résultats présentés montrent que l'activité RT mesurée dans le système de l'invention est jusqu'à 30 fois supérieure à celle mesurée dans les mêmes conditions dans le surnageant des cellules d'encapsidation GP + envAm12, ce qui confirme les propriétés avantageuses de la présente invention pour la production de particules rétrovirales (Tableau 1). De plus, l'activité RT élevée mesurée persiste au-delà d'une semaine dans les cellules co-infectées, en comparaison des procédures de l'art antérieur dans lesquelles l'activité RT décroit après 48 à 96 heures (Vile et al., Br. Med. Bull. 51 (1995) 12), montrant encore les capacités avantageuses de la présente invention.

### 6.3. Encapsidation des ARNs TK dans les particules rétrovirales

II s'agit de détecter et de quantifier les ARNs TK⁺ encapsidés dans les particules virales produites après co-infection de cellules W162 avec AdGAG/POL et AdTK/ENV à différentes moi.

Les surnageants de culture sont prélevés 72 heures après l'infection puis l'ARN est extrait des particules rétrovirales selon la technique décrite par Murdoch et al. [1997, Gene Ther. 4: 744-749]. Les ARNs sont déposés sur une membrane nylon puis hybridés avec une sonde TK radioactive selon les protocoles décrits par Maniatis T. et al. ["Molecular Cloning, a Laboratory Manual", Cold Spring Harbor Laboratory, Cold Spring Harbor, N.Y., 1982].

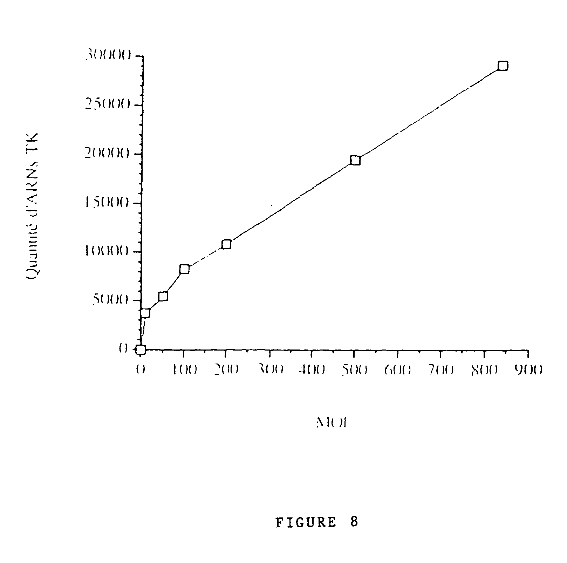

Les résultats obtenus sont présentés sur la Figure 8 et démontrent la présence d'ARNs TK⁺ dans les particules virales produites. La quantité d'ARNs TK⁺ encapsidés est proportionnelle à la moi de la co-infection adénovirale.

La présence d'ARN TK⁺ dans les particules rétrovirales signifie que le mécanisme d'encapsidation est fonctionnel et que les particules virales sont susceptibles de transmettre le génome recombinant dans les cellules infectées.

### 6.4. Titration des particules rétrovirales recombinantes infectieuses

II s'agit d'estimer la quantité de particules rétrovirales infectieuses produites après co-infection de cellules W162 avec AdGAG/POL et AdTK/ENV.

Le surnageant des cellules co-infectées est prélevé 48 heures après l'infection. Des dilutions du surnageant sont déposées sur des cellules NIH3T3 (fibroblastes prélevés dans la queue de souris NIH). Le nombre de cellules NIH3T3 TK⁺ est déterminé par cytométrie de flux (FACS) 48 heures post-infection. Les cellules sont récoltées et fixées 30 min avec du formaldehyde 4% puis lysées 10 min avec du triton 0,2%. Les cellules sont incubées avec un anticorps IgG lapin anti-TK (pAbTK41) dilué au 1/250, lavées (tampon Power-Block 1X, Biogenex) puis marquées avec un anticorps anti-IgG lapin couplé biotine dilué au 1/250 (Sigma). Après lavage, les cellules sont incubées 1 heure avec de la streptavidine-FITC diluée au 1/500 (Amersham) puis analysées par cytométrie de flux.

Le titre rétroviral moyen sur NIH3T3 est de 1,3 x 10⁵ U.l.(unité infectieuse)/ml. Ce résultat indique ainsi que les rétrovirus recombinants produits sont fonctionnels c'est à dire infectieux et capables de transmettre la cassette d'expression TK à des cellules cibles. Le titre viral calculé démontre une productivité de particules rétrovirales infectieuses équivalente aux lignées cellulaires d'encapsidation actuellement disponibles.

### Exemple 7. Présence et expression à long-terme du transgène tk in vitro:

Cet exemple a pour but de démontrer la présence et l'expression de la cassette rétrovirale TK dans des cultures à long-terme de cellules co-infectées avec AdTK/ENV et AdGAG/POL. Des cellules non infectées et des cellules infectées par AdTK/ENV constituent des témoins. Deux types cellulaires ont été infectés: W162 et NIH3T3.

### 7.1. Présence d'une cassette rétrovirale TK intégrée dans le génome de cellules infectées in vitro

II s'agit de tester la présence de la cassette rétrovirale TK dans le génome de cellules mises en culture après co-infection avec AdTK/ENV et AdGAG/POL. Rappelons que le ciston *gag-pol* code notamment pour les protéines reverse-transcriptase (RT) et intégrase (IN) nécessaires pour la reverse-transcription puis l'intégration du génome rétroviral dans les cellules infectées.

L'ADN génomique des cellules en culture est extrait (NIH3T3: J17 et J59 p.i.; W162: J52 et J94 p.i.) puis purifié avec le "QlAamp tissue kit" (Qiagen) selon les indications du fournisseur. L'ADN (2 µg) est utilisé comme matrice dans une réaction de PCR avec l'oligonucléotide SEQ ID N°13 correspondant à la séquence complémentaire de l'extrémité 5' de la région U3 et l'oligonucléotide SEQ ID N°14 correspondant à la séquence complémentaire de l'extrémité 3' du gène *tk* décrits ci-dessous.
SEQ ID N°13
   5'-GCTAGCTTAAGTAACGCCATTTTGC-3'
SEQ ID N°14
   5'-GCGCCAGGTCGCATATCGTCGG-3'

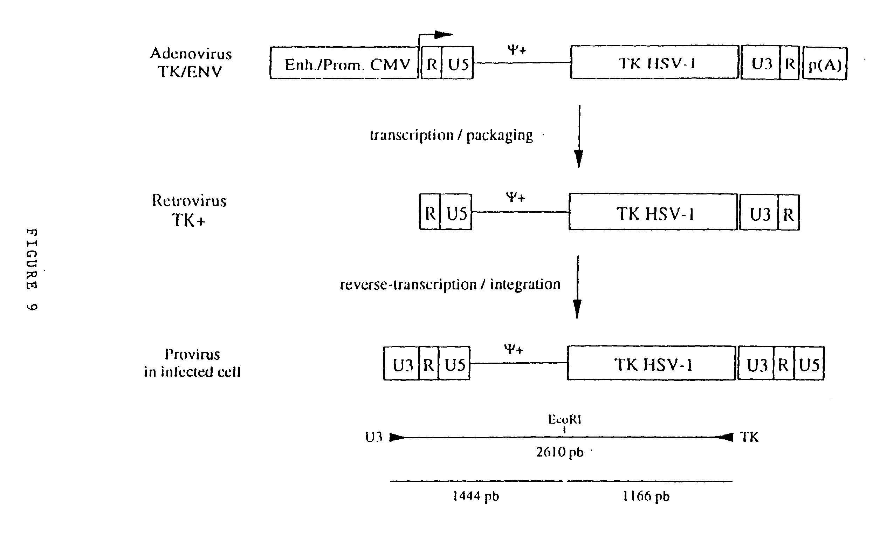

Ces oligonucléotides ont été choisis pour amplifier un fragment de 2610 nt qui peut être coupé (site EcoR1) en deux fragments de 1444 nt et 1166 nt (voir Figure 9). Ce fragment est visualisé par électrophorèse sur gel d'agarose 0,7%.

Pour toutes les dates testées, un fragment PCR ayant le profil attendu (taille + digestion EcoR1) est présent dans les cultures de cellules NIH3T3 et W162 co-infectées avec AdTK/ENV et AdGAG/POL. Il a été vérifié que ce fragment s'hybride spécifiquement avec une sonde complémentaire du gène *tk* (analyse par Southern-blot). Notons que dans notre essai, le signal est plus important dans les cellules NIH3T3. Aucun fragment n'est détecté dans les cultures de cellules non-infectées ou infectées seulement avec AdTK/ENV.

Ces données démontrent que la cassette rétrovirale TK a été transmise via les rétrovirus recombinants produits à des cellules cultivées en présence de cellules co-infectées avec AdTK/ENV et AdGAG/POL. Les dates tardives retenues pour les tests et l'absence de signaux dans les cultures témoins permettent d'affirmer que la cassette TK est intégrée dans le génome des cellules. D'autre part, la présence de la séquence U3 en 5', caractérisée par la possibilité d'amplifier le fragment PCR, signifie que le processus de reverse-transcription est complet.

La co-infection de cellules avec les vecteurs chimériques adéno-rétroviraux AdTK/ENV et Ad GAG/POL permet donc, contrairement à l'infection avec AdTK/ENV seul, l'intégration de la cassette d'expression TK, étape requise pour une expression à long terme de la protéine.

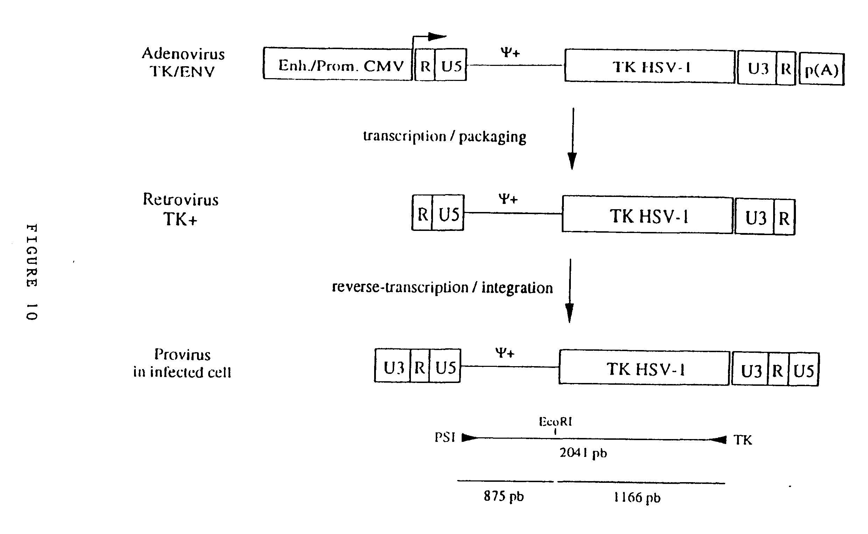

Ces résultats ont été confirmés en utilisant le couple d'oligonucléotides SEQ ID NO: 15 5'-GCTCGTCCGGGATTTGGAGACCC-3' (séquence complémentaire de l'extrémité 5' de la séquence d'encapsidation) et SEQ 14 (Figure 10).

### 7.2. Mesure de la cytotoxicité du gancyclovir (GCV) sur des cellules infectées en culture

II s'agit de tester la présence d'une protéine TK fonctionnelle dans des cellules mises en culture après co-infection avec AdTK/ENV et AdGAG/POL. Pour cela, les cellules sont incubées en présence de gancyclovir (GCV). Rappelons que la thymidine kinase d'HSV-1 transforme les analogues de nucléosides, comme le gancyclovir ou l'acyclovir, en molécules toxiques pour la division cellulaire car celles-ci inhibent la synthèse d'ADN.

Les cellules NIH3T3 (J31 p.i.) et W162 (J69 p.i.) sont incubées en présence de gancyclovir (Roche) dans une gamme de concentrations adaptées (NIH3T3: 0,1, 1, 5 et 10 µM; W162: 1, 5, 10 et 100 µM) pendant 3 à 5 jours. Le nombre de cellules vivantes est déterminé avec le kit "CellTiter AQueous One Solution Cell Proliferation assay" (Promega) selon les indications du fournisseur.

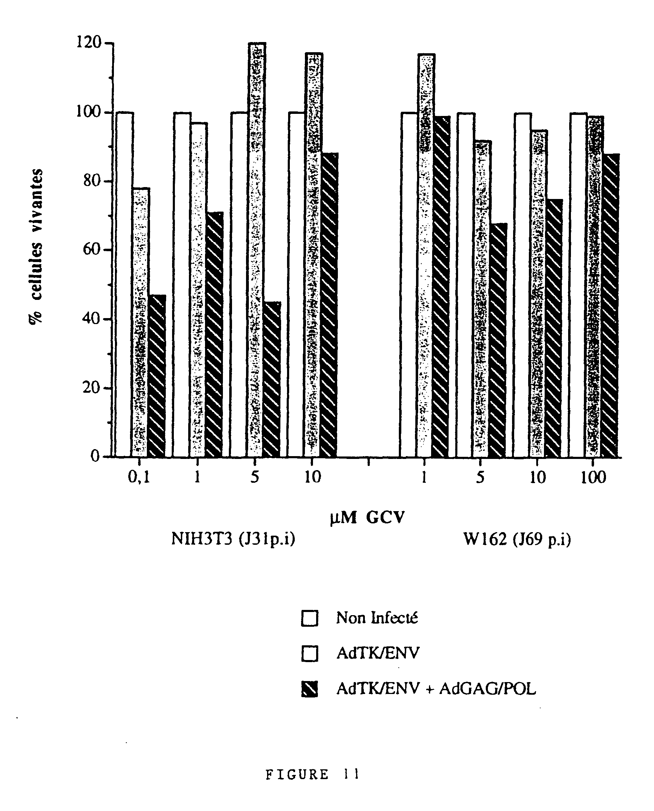

Les résultats obtenus sont présentés sur la Figure 11. Une sensibilité au gancyclovir des cellules est observée dans les cultures co-infectées avec AdTK/ENV et AdGAG/POL. En moyenne, 40% (NIH3T3) à 18% (W162) des cellules sont sensibles au GCV. Cette différence de sensibilité entre les deux types cellulaires peut être corrélée avec la quantité de fragment ADN TK⁺, amplifié par PCR, plus importante dans le cas les cellules NIH3T3 (voir paragraphe 7.2.). Notons que le nombre de cellules sensibles au gancyclovir peut être augmenté par l'effet bystander [Fick, J. et al., 1995, Proc. Natl. Acad. Sci. USA. 92: 11071-110751.

Ces données démontrent que la cassette rétrovirale TK intégrée dans le génome des cellules, mises en culture après co-infection avec AdTK/ENV et AdGAG/POL, code pour une protéine TK fonctionnelle. La co-infection de cellules avec les vecteurs chimériques adéno-rétroviraux AdTK/ENV et Ad GAG/POL permet donc une expression à plus long-terme de la protéine TK que l'infection de cellules avec l'adénovirus AdTK/ENV seul.

### Exemple 8 : Amplification du transgène in vivo

L'efficacité des compositions de l'invention pour la production de rétrovirus et le transfert d'acides nucléiques a été confirmée in vivo, dans des cellules tumorales.

Pour cela, les cellules de la lignée de carcinome d'origine humaine NCI-H460 ont été co-infectées in vitro avec les adénovirus Ad TK/ENV et Ad GAG/POL (MOI de 100 PI/Cellule pour chaque virus), ou avec l'adénovirus Ad TK/ENV seul (MOI de 100 PI/cellule). Une analyse par cytométrie de flux a montré que ces conditions d'infection conduisent à environ 25% de cellules TK-positives. 20 heures après l'infection, 5 x 10⁶ cellules ont été implantées par voie sous-cutanée dans le flanc de souris nude athymiques (n = 4) agées de 4 à 6 semaines. Les animaux ont été sacrifiés 23 jours après l'injection et les tumeurs ont été récoltées et analysées pour la présence et la persistence du transgène. En tant que contrôle, des cellules non infectées ont également été implantées (n = 2).

La présence (et l'amplification) du transgène ont été démontrées par analyse par PCR PSI/TK des tumeurs. Pour cela, l'ADN total a été purifié à partir de 50 mg de tumeur au moyen du kit QlAamp blood (Qiagen). La PCR a été réalisée sur 2 µg d'ADN total en utilisant l'oligonucléotide TK (SEQ ID NO:14) et l'oligonucléotide PSI de séquence 5'-GCTCGTCCGGGATTTGGAGACCC-3' (SEQ ID NO: 16) comme amorces, générant un fragment de 2041 pb à partir du provirus TK et de l'ADN de Ad TK/ENV. 35 cycles d'amplification on été effectués à une température dénaturante de 95°C pendant 45 secondes, une température d'annealing de 65°C pendant 45 secondes , et une température d'élongation de 72°C pendant 4 minutes et 30 secondes. Comme contrôle, une PCR a été réalisée sur chaque échantillon d'ADN en utilisant le jeu human β-Actin Control Amplimer (Clontech). Les produits PCR ont été visualisés sur gel d'agarose 1% par coloration au bromure d'éthidium.

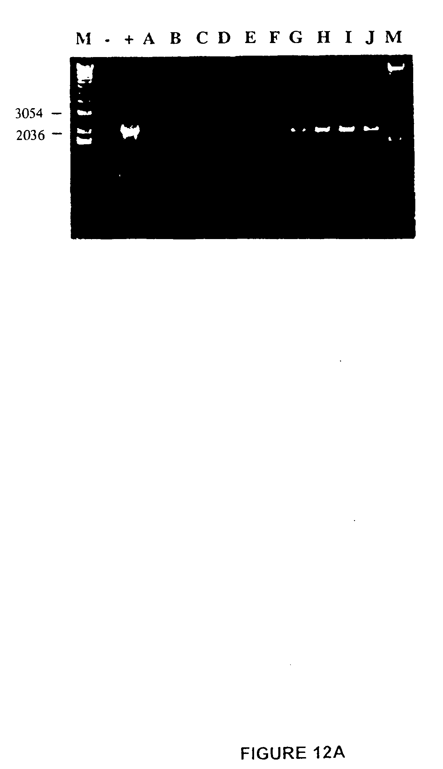

Les résultats obtenus sont présentés sur la figure 12A, et montrent que les tumeurs qui se développent après co-infection avec les adénovirus Ad TK/ENV et Ad GAG/POL (tumeurs G, H, I est J) contiennent un nombre plus élevé de copies du gènes TK que celles ayant reçu seulement Ad TK/ENV (tumeurs C, D, E et F), ou n'ayant reçu aucun virus (tumeurs A et B).

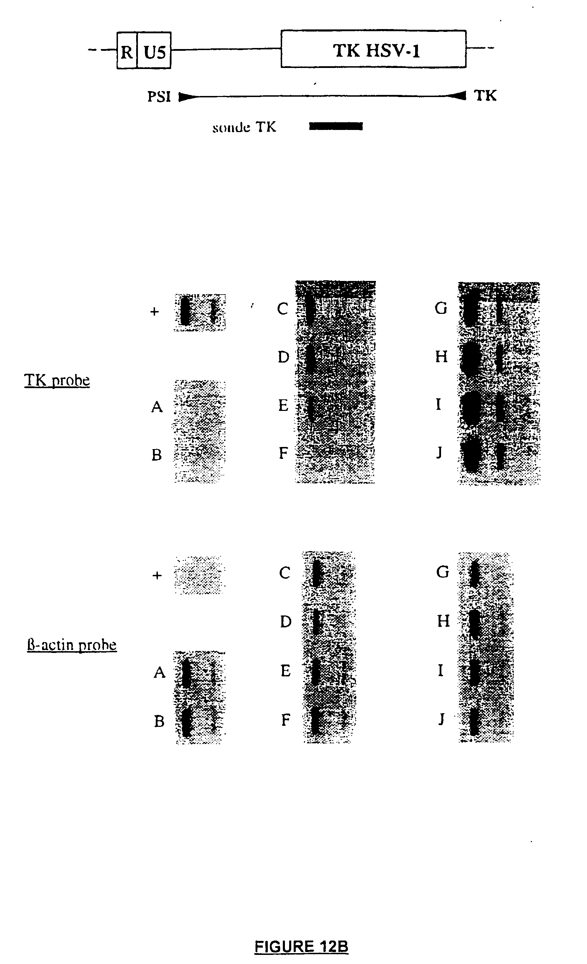

L'amplification du nombre de copies du transgène a été confirmée par analyse quantitative Slot-Blot. Pour cela, 1 µl de chaque produit d'amplification PCR PSI/TK et PCR β-Actin a été chauffé dans 0,4 M NaOH 2,2 mM EDTA à 95°C pendant 2 minutes, et transféré sur membrane de nylon hybond-N+ (Amersham Life Sciences). L'ADN a été fixé et les membranes préhybridées 2 heures à 65°C dans 0,25 M NaH₂PO4-0,25 M Na₂HPO4 - 7% SDS, suivi d'une hybridation pendant la nuit dans les mêmes conditions. Les membranes sont ensuite lavées 2 fois à 65°C, 2 x SSC, 0,1% SDS, 5 minutes ; 2 fois à 65°C, 2 x SSC, 0,1% SDS, 15 minutes, et 2 fois à 65°C, 0,1 x SSC, 0,1% SDS, 30 minutes. La sonde utilisée correspond au gène HSV-1 TK, marquée de manière aléatoire au (α - ³²P) dCTP. Les membranes ont été analysées avec un phosphorimager Fujix Bas 1000 (Fuji Photo Film Co.). Les résultats obtenus sont présentés sur la figure 12B, et montrent une augmentation d'un facteur 10 à 50 du nombre de cellules tumorales contenant le gène TK, en comparaison des tumeurs infectées par Ad TK/ENV seul.

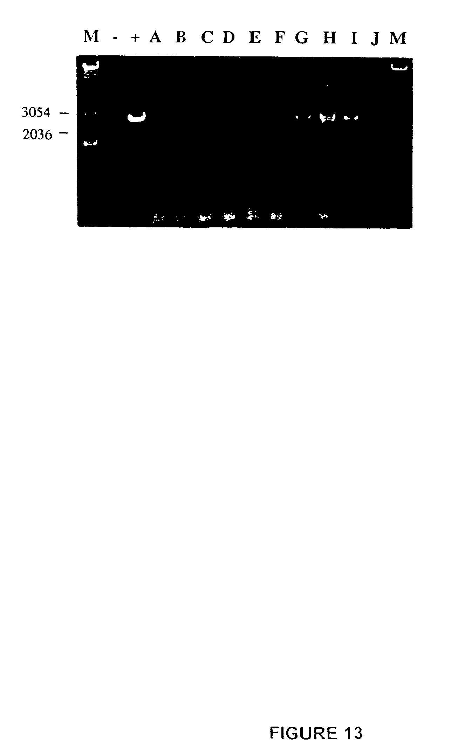

L'intégration du gène TK dans les cellules tumorales co-infectées a été confirmée par analyse PCR (figure 13, linges G-J), alors que le provirus TK n'a pu être détecté dans les cellules infectées par Ad TK/ENV seul (Figure 13, lignes 1 A-F).

Ces résultats montrent donc (i) l'efficacité de l'invention in vivo (ii) l'amplification du transgène au moyen du système de l'invention et (iii) l'intégration du transgène dans le chromosome des cellules cibles in vivo. L'ensemble de ces résultats illustre les propriétés avantageuses de la présente invention pour le transfert d'acides nucléiques dans les cellules et la production de particules rétrovirales infectieuses.

**TABLEAU 1**

| SURNAGEANT DES CELLULES | MOI | ACTIVITE RT |
|---|---|---|
| Non-infectées | - | 1 |
| GP+*env*Am 12 | - | 4.9x10² |
| AdTK/ENV | 200 | 1 |
| AdGAG/POL | 200 | 1.7x10⁵ |
| AdGAG/POL+AdTK/ENV | 10/10 | 1.9x10⁴ |
| AdGAG/POL+AdTK/ENV | 50/50 | 6.4x10⁴ |
| AdGAG/POL+AdTK/ENV | 100/100 | 1.4x10⁵ |
| AdGAG/POL+AdTK/ENV | 200/200 | 1.7x10⁵ |
| AdGAG/POL+AdTK/ENV | 300/300 | 1.7x10⁵ |
| AdGAG/POL+AdTK/ENV | 500/500 | 1.5x10⁵ |

## Revendications

1. Composition comprenant l'ensemble des éléments génétiques nécessaires à la constitution d'une particule rétrovirale, incorporés dans deux adénovirus recombinants avec la répartition particulière suivante des éléments génétiques rétroviraux, ladite composition comprenant :
- un premier adénovirus recombinant comprenant, incorporé dans son génome, un ou plusieurs acides nucléiques codant pour les protéines rétrovirales gag et pol, et
- un deuxième adénovirus recombinant comprenant, incorporés dans son génome, un acide nucléique codant pour une protéine d'enveloppe, et un acide nucléique comprenant, entre deux régions LTR, une séquence d'encapsidation rétrovirale et une séquence nucléique d'intérêt,
lesdits adénovirus recombinants comprenant des délétions affectant tout ou une partie fonctionnelle des régions E1, E4 et E3.

2. Composition selon la revendication 1, **caractérisée en ce que** les protéines gag et pol sont des protéines de rétrovirus choisis parmi MoMLV, ALV, BLV, MMTV, et RSV.

3. Composition selon la revendication 1, **caractérisée en ce que** la protéine d'enveloppe est une protéine virage permettant aux particules rétrovirales d'infecter les cellules humaines.

4. Composition selon la revendication 3, **caractérisée en ce que** la protéine d'enveloppe est une protéine d'enveloppe d'un virus GALV, A4070, RD114, VSV-G ou de la rage.

5. Composition selon la revendication 1, **caractérisée en ce que** la ou les régions LTR sont des régions LTR rétrovirales complètes ou des sous-domaines permettant de reconstituer des LTRs complets après activité reverse transcriptase.

6. Composition selon la revendication 5, **caractérisée en ce que** le LTR 5' est dépourvu du domaine U3 et le LTR 3' est dépourvu du domaine U5.

7. Composition selon la revendication 1 **caractérisée en ce que** les éléments génétiques permettent la constitution d'une particule de lentivirus.

8. Composition selon la revendication 1, **caractérisée en ce que** le ou les adénovirus recombinants sont dépourvus en plus de tout ou partie des gènes codant pour les fonctions tardives de l'adénovirus.

9. Utilisation d'une composition selon l'une des revendications 1 à 8 pour la production in vitro ou ex vivo de particules rétrovirales.

10. Utilisation d'une composition selon l'une des revendications 1 à 8 pour la préparation d'un médicament destiné à la production de particules rétrovirales in vivo.

11. Cellule modifiée par une composition selon l'une des revendications 1 à 8 pour la préparation d'un médicament destiné au transfert d'acides nucléiques dans les cellules in vivo.

12. Procédé de production de particules rétrovirales in vitro, comprenant l'incubation de cellules en présence d'une composition selon l'une des revendications 1 à 8.

13. Procédé selon la revendication 12, **caractérisé en ce que** les cellules sont des cellules d'origine humaine.

## Claims

1. A composition comprising the whole set of genetic elements required for constituting a retroviral particle, incorporated in two recombinant adenoviruses with the following particular distribution of the retroviral genetic elements, said composition comprising :
- a first recombinant adenovirus comprising, incorporated in its genome, one or several nucleic acids coding for retroviral gag and pol proteins, and
- a second recombinant adenovirus comprising, incorporated in its genome, a nucleic acid coding for an envelope protein, and a nucleic acid comprising, between two LTR regions, a retroviral packaging sequence and a nucleic acid sequence of interest,
said recombinant adenoviruses comprising deletions affecting all or a functional part of the E1, E4 and E3 regions.

2. The composition according to claim 1, **characterized in that** the gag and pol proteins are proteins from retroviruses selected from MoMLV, ALV, BLV, MMTV and RSV.

3. The composition according to claim 1, **characterized in that** the envelope protein is a viral protein allowing retroviral particles to infect human cells.

4. The composition according to claim 3, **characterized in that** the envelope protein is an envelope protein from a GALV, A4070, RD114, VSV-G or rabies virus.

5. The composition according to claim 1, **characterized in that** the LTR region(s) is (are) complete retroviral LTR regions or subdomains allowing reconstitution of complete LTRs after reverse transcriptase activity.

6. The composition according to claim 5, **characterized in that** the 5' LTR is deleted for the U3 domain and the 3' LTR is deleted for the U5 domain.

7. The composition according to claim 1, **characterized in that** the genetic elements allow the constitution of a lentivirus particle.

8. The composition according to claim 1, **characterized in that** the recombinant adenovirus(es) is (are) further defective for all or part of the genes encoding the adenoviral late functions.

9. Use of a composition according to one of claims 1 to 8 for the production of retroviral particles *in vitro* or *ex vivo.*

10. Use of a composition according to one of claims 1 to 8 for the preparation of a drug intended for the production of retroviral particles *in vivo.*

11. A cell modified by a composition according to one of claims 1 to 8 for the preparation of a drug intended for transferring nucleic acids into cells *in vivo.*

12. A method for producing retroviral particles *in vitro,* comprising incubating cells in the presence of a composition according to one of claims 1 to 8.

13. The method according to claim 12, **characterized in that** the cells are cells of human origin.

## Patentansprüche

1. Zusammensetzung, umfassend die gesamten genetischen Elemente, die für die Bildung eines retrovialen Partikels erforderlich sind und in zwei rekombinanten Adenoviren mit der folgenden speziellen Verteilung der retroviralen genetischen Elemente eingebaut sind, wobei besagte Zusammensetzung umfasst:
- ein erstes rekombinantes Adenovirus, umfassend, eingebaut in seinem Genom, eine oder mehrere Nukleinsäuren, die für die retroviralen Proteine gag und pol codieren, und
- ein zweites rekombinantes Adenovirus, umfassend, eingebaut in seinem Genom, eine Nukleinsäure, die für ein Hüllprotein codiert, und eine Nukleinsäure, die zwischen zwei LTR-Regionen eine retrovirale Enkapsidierungssequenz und eine Nukleinsäuresequenz von Interesse umfasst,
wobei besagte rekombinante Adenoviren Deletionen umfassen, die die gesamten oder einen funktionellen Teil der Regionen E1, E4 und E3 beeinträchtigen.

2. Zusammensetzung gemäß Anspruch 1, **dadurch gekennzeichnet, dass** die Proteine gag und pol Proteine von Retroviren sind, die aus MoMLV, ALV, BLV, MMTV und RSV ausgewählt sind.

3. Zusammensetzung gemäß Anspruch 1, **dadurch gekennzeichnet, dass** das Hüllprotein ein virales Proteine ist, das den retroviralen Partikeln eine Infektion humaner Zellen erlaubt.

4. Zusammensetzung gemäß Anspruch 3, **dadurch gekennzeichnet, dass** das Hüllprotein ein Hüllprotein vom GALV-, A4070-, RD114-, VSV-G-Virus oder vom Rabiesvirus ist.

5. Zusammensetzung gemäß Anspruch 1, **dadurch gekennzeichnet, dass** die LTR-Region oder LTR-Regionen vollständige retrovirale LTR-Regionen oder Subdomänen sind, die eine Generierung vollständiger LTRs nach Aktivität der reversen Transkriptase erlauben.

6. Zusammensetzung gemäß Anspruch 5, **dadurch gekennzeichnet, dass** die 5'-LTR, ohne die U3-Domäne und die 3'-LTR, ohne die U5-Domäne vorliegt.

7. Zusammensetzung gemäß Anspruch 1, **dadurch gekennzeichnet, dass** die genetischen Elemente die Bildung eines Lentivirus-Partikels erlauben.

8. Zusammensetzung gemäß Anspruch 1, **dadurch gekennzeichnet, dass** das rekombinante Adenovirus oder die rekombinanten Adenoviren außerdem ohne die gesamten oder einen Teil der Gene sind, die für die späten Funktionen des Adenovirus codieren.

9. Verwendung einer Zusammensetzung gemäß einem der Ansprüche 1 bis 8 zur in vitro oder ex vivo Produktion retroviraler Partikel.

10. Verwendung einer Zusammensetzung gemäß einem der Ansprüche 1 bis 8 zur Herstellung eines Medikaments für die in vivo Produktion retroviraler Partikel.

11. Zelle, die mithilfe einer Zusammensetzung gemäß einem der Ansprüche 1 bis 8 verändert ist, zur Herstellung eines Medikaments für die in vivo Übertragung von Nukleinsäuren in die Zellen.

12. Verfahren zur in vitro Produktion retroviraler Partikel, umfassend die Inkubation von Zellen in Gegenwart einer Zusammensetzung gemäß einem der Ansprüche 1 bis 8.

13. Verfahren gemäß Anspruch 12, **dadurch gekennzeichnet, dass** die Zellen humanen Ursprungs sind.
